# EUROPEAN PATENT SPECIFICATION

(11) **EP 2 382 996 B1**
(45) Date of publication and mention of the grant of the patent: **14.01.2015**
(21) Application number: 11172230.2
(22) Date of filing: 17.02.2006
(51) Int. Cl.: A61K 47/48, C07K 5/04, A61K 9/127, A61K 47/44, C07K 7/06, C07K 5/08, C07K 5/10

(54) **Lipid film structure containing a polyoxyalkylene chain-containing lipid derivative.**
Lipidfilmstruktur enthaltend ein Polyoxyalkylenkettenhaltiges Lipidderivat
Structure de film lipidique contenant un dérivé de lipides contenant une chaîne de polyoxyalkylène

(30) Priority: 18.02.2005 JP 2005043196
(43) Date of publication of application: 02.11.2011
(62) Divisional of application: 06714495.6
(73) Proprietor: The University of Tokushima, Tokushima-shi, Tokushima 770-8501 (JP); NOF CORPORATION, Tokyo 150-6019 (JP)
(72) Inventor: KIWADA, Hiroshi, Tokushima-shi, Tokushima 7708505 (JP); ISHIDA, Tatsuhiro, Tokushima-shi, Tokushima 7708505 (JP); ITO, Chika, Kawasaki-shi, Kanagawa 2100865 (JP); KUBO, Kazuhiro, Kawasaki-shi, Kanagawa 2100865 (JP); SAKANOUE, Kenji, Kawasaki-shi, Kanagawa 2100865 (JP); KIKUCHI, Hiroshi, Tokyo, 135-0046 (JP)
(74) Representative: Duckworth, Timothy John

(56) References cited:
- WO-A-01/00247
- US-A- 5 213 804
- US-A1- 2003 171 262
- KIRPOTIN ET AL: "Liposomes with detachable polymer coating: destabilization and fusion of dioleoylphosphatidylethanolamine vesicles triggered by cleavage of surface-grafted poly(ethylene glycol)", FEBS LETTERS, ELSEVIER, AMSTERDAM, NL, vol. 388, no. 2/03, 1 January 1996 (1996-01-01), pages 115-118, XP002080855, ISSN: 0014-5793

## Description

### Technical Field

The present invention relates to a lipid membrane structure containing a polyoxyalkylene chain-containing lipid derivative and a polyoxyalkylene chain-containing lipid derivative.

### Background Art

Drug delivery systems (DDS) are being developed for the purposes of reducing the adverse reactions of bioactive substances such as drugs, and improving the selective delivery of drugs to target sites. For example, a method of modifying a polypeptide in a protein preparation and a method of including a drug in a liposome or a microparticle such as a polymeric micelle using a copolymer of a hydrophilic polymer and a hydrophobic polymer are available. Early liposome preparations were problematic in that they were likely to be captured by the Reticulo-Endothelial System (hereinafter referred to as "RES") in the liver, and spleen due to poor retention in blood when intravenously administered. For this reason, the presence of RES had long been a major barrier to utilizing microparticle pharmaceutical carriers as targeting type preparations for delivering a pharmaceutical to an organ other than RES and sustained-release preparations that allow preparation retention in blood for a long time to control the release of a pharmaceutical.

To solve these problems, with the intent to avoid the capture by RES, a liposome conferred with microcirculatory by modifying the membrane surface with a glycolipid, glycoprotein, or polyethylene glycol lipid has been proposed (Pamphlet for International Patent Publication No. 91/05546). According to the liposome, it is possible to improve the retention in blood, and to allow liposome particles to selectively permeate the vascular cells of cancer cells by means of an EPR effect (Enhanced Permeability and Retention Effect), so as to accumulate the drug near the cancer cells. However, this liposome is problematic in that the drug included therein is not quickly released.

Recently, it was reported that a liposome using a functional phospholipid is capable of actively and efficiently delivering a drug to a target site, rather than achieving the above-described passive delivery of a drug to a target site (Japanese Patent Kokai Publication No. HEI-04-346918). Also reported was a method comprising binding a carboxy alkylpullulan polyalcohol to the active ingredient of an antitumor agent via a spacer consisting of amino acids to form a drug complex, in order to selectively transfer the antitumor agent to a target site (Japanese Patent Kokai Publication No. HEI-11-092405).

### Disclosure of the Invention

However, according to these methods, drug retention in blood, and transportability to a target site are improved, but problems such as activity reduction arise for some drugs because a polysaccharide are covalently bound directly to the drug.

The present invention has been developed in view of these circumstances, and it is an object of the invention to provide a novel lipid derivative and lipid membrane structure that exhibit excellent retention in blood and selective delivery to a target site when applied to a drug delivery system.

The present inventors conducted diligent investigations with the aim of accomplish the above-described object, and found that when a lipid derivative incorporating a polyoxyalkylene chain was obtained and a lipid membrane structure comprising the same was applied to a drug delivery system, it became possible to retain the drug in blood for a long time, and to regulate the blood retention because of efficient cleavage of the polyoxyalkylene chain at the target site. The present inventors conducted further extensive investigations, and found that upon cleavage of the polyoxyalkylene chain, the stability of the lipid membrane structure decreases and the bioactive substance included therein is released, so that the bioactive substance can be efficiently delivered and released to the target site. Based on these findings, the present inventors developed the present invention.

Accordingly, the present invention encompasses the items shown below.
[1.] A lipid membrane structure having a lipid membrane comprising a polyoxyalkylene chain-containing lipid derivative, which is represented by the following formula (1): 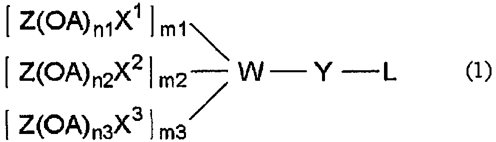 wherein
   L is a residue derived from an aliphatic hydrocarbon having 8 to 24 carbon atoms, which comprises at least one kind of group out of an amino group, a hydroxyl group, a carboxyl group and a maleimide group, or a lipid selected from the group consisting of a glycerolipid, a sphingolipid and a sterol,
   Y is a divalent group comprising a group selected from the group consisting of -CONH-, -NHCO-, -OCONH-, -NHOCO-, -COO-, - OOC-, -CH₂NH-, -NHCH₂-, -S-CH<, >CH-S-, and -O-,
   W is a polyamino acid residue comprising 2 to 6 glycine residues, which is represented by Gly-Gly, Gly-Gly-Gly, Gly-Gly-Gly-Gly, Gly-Gly-Gly-Gly-Gly, or Gly-Gly-Gly-Gly-Gly-Gly, X¹ is a divalent group comprising -NHCO- or -OOC-,
   X² is a divalent group comprising a group selected from the group consisting of -OCONH-, -CONH-, -CH₂NH-, and -NHC(O)NH-, X³ is a divalent group comprising a group selected from the group consisting of >CH-S-, -NHCOO-, -COO-, -COS-, and -O-,
   OA is an oxyalkylene group having 2 to 4 carbon atoms,
   Z is a hydrogen atom or a methyl group,
   each of m1, m2 and m3 independently represents a positive number of 0 to 5 that satisfies the requirement of 1 ≤ m1+m2+m3 ≤ 9, and
   each of n1, n2 and n3 independently represents a positive number of 4 to 800 that satisfies the requirement of 4 ≤
   (n1Xm1)+(n2Xm2)+(n3Xm3) ≤ 2000, and a phosphatidylethanolamine as a constituent of the lipid membrane.
[2.] The structure described in [1] above, wherein X¹ is a divalent group comprising -OOC-, X² is a divalent group comprising -OCONH- or -CH₂NH-, and X³ is a divalent group comprising >CH-S- or -O-.
[3.] The structure described in [2] above, wherein m1 is 0.
[4.] The structure described in any one of [1] to [3] above, wherein OA is an oxyethylene group.
[5.] The structure described in any of [1] to [4] above, wherein each of m1, m2 and m3 is a positive number that satisfies the requirement of 1 ≤ m1+m2+m3 ≤ 6, and each of n1, n2 and n3 is a positive number that satisfies the requirement of 40≤ (n1Xm1)+(n2Xm2)+(n3Xm3) ≤ 1000.
[6.] The structure described in any of [1] to [5] above, wherein each of m1, m2 and m3 is a positive number that satisfies the requirement of 2 ≤ m1+m2+m3 ≤ 6.
[7.] The structure described in any of [1] to [6] above, wherein each of m1 and m3 is 0, m2 is 1, X² is a divalent group comprising -OCONH-, and Y is a divalent group comprising -NHCO-.
[8.] The structure described in any of [1] to [7] above, wherein L is a residue derived from a glycerophospholipid.
[9.] The structure described in any of [1] to [8] above, wherein W is a polyamino acid residue comprising a dipeptide represented by Phe-Gly, Leu-Gly, or Tyr-Gly, and/or W is a polyamino acid residue comprising a Lys residue, an Arg residue, a Cys residue, an Asp residue, a Glu residue or a Ser residue.
[10.] The structure described in any of [1] to [9] above, wherein the content ratio of the polyoxyalkylene chain-containing lipid derivative is 0.1 to 25% by weight relative to the total weight of the lipid membrane structure.
[11.] The structure described in any of [1] to [10] above, wherein the phosphatidylethanolamine is dioleoylphosphatidylethanolamine.
[12.] The structure described in any of [1] to [11] above, which is in the form of a liposome.
[13.] The structure described in [12] above, comprising a bioactive substance.
[14.] The structure described in [13], wherein the bioactive substance is released at pH 6 or below.
[15.] The structure described in [1] above, wherein L is a dioleoylphosphatidylethanolamine residue, and the phosphatidylethanolamine is dioleoylphosphatidylethanolamine.
[16.] A polyoxyalkylene chain-containing lipid derivative, which is represented by the following formula (1): 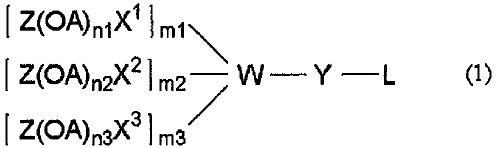 wherein
   L is a residue derived from an aliphatic hydrocarbon having 8 to 24 carbon atoms, which comprises at least one kind of group out of an amino group, a hydroxyl group, a carboxyl group and a maleimide group, or a lipid selected from the group consisting of a glycerolipid, a sphingolipid and a sterol,
   Y is a divalent group comprising a group selected from the group consisting of -CONH-, -NHCO-, -OCONH-, -NHOCO-, -COO-, - OOC-, -CH₂NH-, -NHCH₂-, -S-CH<, >CH-S-, and -O-,
   W is a polyamino acid residue comprising 2 to 6 glycine residues, which is represented by Gly-Gly, Gly-Gly-Gly, Gly-Gly-Gly-Gly, Gly-Gly-Gly-Gly-Gly, or Gly-Gly-Gly-Gly-Gly-Gly,
   X¹ is a divalent group comprising -NHCO- or -OOC-,
   X² is a divalent group comprising a group selected from the group consisting of -OCONH-, -CONH-, -CH₂NH-, and -NHC(O)NH-,
   X³ is a divalent group comprising a group selected from the group consisting of >CH-S-, -NHCOO-, -COO-, -COS-, and -O-,
   OA is an oxyalkylene group having 2 to 4 carbon atoms,
   Z is a hydrogen atom or a methyl group,
   each of m1, m2 and m3 independently represents a positive number of 0 to 5 that satisfies the requirement of 1 ≤ m1+m2+m3 ≤ 9, and
   each of n1, n2 and n3 independently represents a positive number of 4 to 800 that satisfies the requirement of 4 ≤ (n1Xm1)+(n2Xm2)+(n3Xm3) ≤ 2000.

### Brief Description of the Drawings

Figure 1 is a drawing showing the ratios of included fluorescent dye released from the liposomes of Examples 5 to 7 and Comparative Example 1 versus pH.
Figure 2 is a drawing showing the stability of the liposome of Example 6 in serum.
Figure 3 is a drawing showing the ratio of included fluorescent dye released from the liposomes of Examples 5 to 6 and Comparative Examples 2 to 3 after papain treatment versus pH.
Figure 4 is a drawing showing the sensitivity of the liposome of Example 8 to cathepsin B and the disintegratability of the liposome.
Figure 5 is a drawing showing the blood kinetics of the liposomes including an anticancer agent (doxorubicin) of Example 9 and Comparative Examples 4 to 5 and the anticancer agent-retention rates of the liposomes.
Figure 6 is a drawing showing the antitumor effect of the liposome including an anticancer agent (doxorubicin) of Example 9.

### Best Mode for Embodying the Invention

The present invention is hereinafter described in detail with reference to preferred embodiments thereof.

The lipid membrane structure having a lipid membrane of the invention comprises a polyoxyalkylene chain-containing lipid derivative (hereinafter sometimes simply referred to as "lipid derivative") which is represented by the formula (1) above.

In the formula (1) above, L is a lipid residue. The lipid residue is a residue derived from an aliphatic hydrocarbon having 8 to 24 carbon atoms comprising at least one kind of group out of an amino group, a hydroxyl group, a carboxyl group and a maleimide group, or a lipid selected from the group consisting of a glycerolipid, a sphingolipid or a sterol. As mentioned herein, "a residue" in the present invention refers to a group other than the amino group, hydroxyl group, carboxyl group or maleimide group contained in the lipid structure.

The residue derived from an aliphatic hydrocarbon having 8 to 24 carbon atoms comprising at least one kind of group out of an amino group, a hydroxyl group, a carboxyl group and a maleimide group may be saturated or unsaturated, and may be linear, branched or cyclic. The carbon number thereof is preferably 12 to 20. As examples of the residue derived from an aliphatic hydrocarbon, octyl group, nonyl group, decyl group, undecyl group, dodecyl group, tridecyl group, tetradecyl group, pentadecyl group, hexadecyl group, heptadecyl group, octadecyl group, isostearyl group, octadecenyl group, octadecadienyl group, nonadecyl group, eicosyl group, docosyl group, dococenyl group, and tetracosyl group can be mentioned.

As examples of the aliphatic hydrocarbon having 8 to 24 carbon atoms comprising at least one kind of group out of an amino group, a hydroxyl group, a carboxyl group and a maleimide group, a fatty acid, an aliphatic amine, an aliphatic alcohol, and derivatives thereof can be mentioned. Specifically, as the fatty acid, caprylic acid, capric acid, lauric acid, myristic acid, palmitic acid, stearic acid, isostearic acid, oleic acid, elaidic acid, linoleic acid, linolenic acid, arachic acid, and arachidonic acid can be mentioned, and the fatty acid is preferably a monocarboxylic acid. As the aliphatic amine, octylamine, decylamine, dodecylamine, tetradecylamine, hexadecylamine, octadecylamine, octadecenylamine, and eicosylamine can be mentioned, and aliphatic monoamines thereof are suitable. As the aliphatic alcohol, octyl alcohol, decyl alcohol, dodecyl alcohol, tetradecyl alcohol, hexadecyl alcohol, octadecyl alcohol, octadecenyl alcohol, and eicosyl alcohol can be mentioned, and aliphatic monoalcohols thereof are suitable.

An aliphatic amine or an aliphatic alcohol can also be used as the lipid comprising a maleimide group after being reacted with a divalent reagent like (e0) shown below.

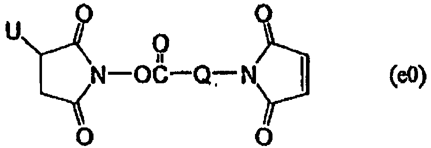

In the formula above, Q is a hydrocarbon group having 1 to 9 carbon atoms (e.g., alkylene group), and U is a hydrogen atom or -SO₃Na.

As the glycerolipid, a glycerophospholipid, and a diacylglycerol can be mentioned. As the glycerophospholipid, phosphatidylethanolamine, phosphatidic acid, phosphatidylglycerol, and phosphatidylserine can be mentioned. The carbon number of each of a plurality of acyl groups present in the glycerophospholipid is independently preferably 8 to 24, more preferably 12 to 20, particularly preferably 18. The acyl groups may be identical or different, may be saturated or unsaturated, and may be linear or branched. The choice of acyl group is not particularly limited; normally, an acyl group derived from a fatty acid can be suitably used. Specifically, acyl groups derived from fatty acids such as caprylic acid, capric acid, lauric acid, myristic acid, palmitic acid, palmitoleic acid, stearic acid, isostearic acid, oleic acid, linoleic acid, arachic acid, behenic acid, erucic acid, and lignoceric acid can be mentioned. When the above-described carbon number exceeds 24, the dispersibility in aqueous phase is poor, and the reactivity tends to decrease. When the above-described carbon number is less than 8, the crystallinity in the purification step is poor, and the purity of the desired product tends to decrease. When a plurality of amino groups, hydroxyl groups, carboxyl groups, or maleimide groups are present in the lipid structure of the glycerolipid residue, at least one kind out of amino groups, hydroxyl groups, carboxyl groups and maleimide groups may remain in the residue.

As the sphingolipid, sphingophospholipids represented by sphingomyelin, and sphingoglycolipids can be mentioned. Ceramide, and sphingosine that are constituents thereof include derivatives thereof. As the derivatives, ceramide phosphorylethanolamine, ceramide phosphorylglycerol, and ceramide phosphorylglycerol phosphate can be mentioned. As the fatty acid that binds to sphingosine, caprylic acid, capric acid, lauric acid, myristic acid, palmitic acid, palmitoleic acid, stearic acid, isostearic acid, oleic acid, linoleic acid, arachic acid, behenic acid, erucic acid, and lignoceric acid can be mentioned.

As examples of the sterol, cholesterol, and cholestanol can be mentioned.

Y is a divalent group comprising -CONH-, -NHCO-, -OCONH-, -NHOCO-, -COO-, -OOC-, -CH₂NH-, -NHCH₂-, -S-CH<, >CH-S-, or -O-. Preferably, Y is a divalent group comprising -CONH-, -NHCO-, - OCONH-, -NHOCO-, -COO-, -OOC-, -NHCH₂-, or -S-CH<. As such, Y is, for example, a group formed by a reaction of the amino group, hydroxyl group, carboxyl group or maleimide group of a lipid and the amino group, carboxyl group, hydroxyl group or thiol group of a polyamino acid, and functions as the binding group for L and W in the formula (1). Y may have a hydrocarbon group, as long as it comprises the above-described group, and is not particularly limited. Hereinafter, divalent groups represented by Y are specifically described.

For example, when the lipid is phosphatidylethanolamine, -CONH- formed by a reaction of the amino group of the lipid and the C-terminal carboxyl group of the polyamino acid or, when the polyamino acid comprises a glutamic acid residue or an aspartic acid residue, a carboxyl group therein; or - CO(CH₂)₂CONH- or -CO(CH₂)₃CONH- formed by a reaction of the lipid incorporating a carboxyl group introduced by a reaction with a dibasic acid anhydride such as succinic anhydride or glutaric anhydride and the N-terminal α-amino group of the polyamino acid or, when the polyamino acid comprises a basic amino acid residue such as lysine, an ε-amino group therein; and a divalent group represented by the following formula (a1), formed by a reaction of the lipid incorporating a maleimide group introduced by a reaction with a reagent represented by the above-described (e0) and, when the polyamino acid comprises a cysteine residue, a thiol group therein, can be mentioned. In the formula below, Q has the same definition as that shown above.

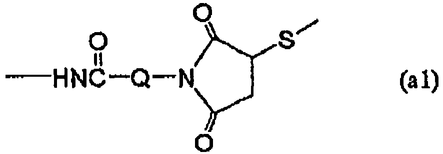

Also, for example, when the lipid is cholesterol or diacylglycerol, -COO- formed by a reaction of the terminal hydroxyl group of the lipid with the C-terminus of the polyamino acid or, when the polyamino acid comprises a glutamic acid residue or an aspartic acid residue, a carboxyl group therein; and -OCONH- formed by a reaction of the above-described terminal hydroxyl group carbonated with a p-nitrophenyl carbonate with the N-terminal α-amino group of the polyamino acid or, when the polyamino acid comprises a basic amino acid residue such as lysine, a ε-amino group therein, can be mentioned. Also, -CH₂CH₂NHCO- or - CH₂CH₂CH₂NHCO- formed by a reaction of the above-described terminal hydroxyl group converted to propylamine or ethylamine with the C-terminal carboxyl group of the polyamino acid or, when the polyamino acid comprises a glutamic acid residue or an aspartic acid residue, a carboxyl group therein; and -S-CH< formed by a reaction of the above-described lipid incorporating a maleimide group introduced thereto with, when the polyamino acid comprises a cysteine residue, a thiol group therein, can be mentioned.

W functions as a group undergoing cleavage by a hydrolase in vivo. The hydrolase is not particularly limited, as long as it is, for example, an enzyme, protease present in the vicinity of tumor cells and inflammatory portions. For example, the hydrolase may be an endopeptidase such as cathepsin, papain, pepsin, trypsin, and chymotrypsin. W is a polyamino acid residue comprising 2 to 6 glycine residues, represented by Gly-Gly, Gly-Gly-Gly, Gly-Gly-Gly-Gly (SEQ ID NO:1), Gly-Gly-Gly-Gly-Gly (SEQ ID NO:2), or Gly-Gly-Gly-Gly-Gly-Gly (SEQ ID NO:3). A polyamino acid residue comprising Gly-Gly-Phe-Gly (SEQ ID NO:4), Gly-Phe-Gly-Gly (SEQ ID NO:5), Phe-Gly-Gly-Gly (SEQ ID NO:6), Phe-Phe-Gly-Gly (SEQ ID NO:7), Gly-Gly-Gly-Phe-Gly (SEQ ID NO:8), Gly-Gly-Phe-Phe (SEQ ID NO:9), or Gly-Gly-Gly-Phe (SEQ ID NO:10) can also be mentioned.

Upon cleavage of the polyamino acid residue represented by W by an enzyme, the polyoxyalkylene chain on the surface of a lipid membrane structure such as liposome is eliminated. Thereby, the lipid membrane structure is exposed to the environment and destroyed, and the bioactive substance is released in the body. For example, when a liposome including a bioactive substance, and formulated with.the lipid derivative of the present invention, is administered into blood, upon reach of the liposome in the vicinity of a tumor site, the polyamino acid residue undergoes hydrolysis by peptidase, resulting in the elimination of the polyoxyalkylene chain and the disappearance of the hydrated layer on the liposome surface. Thereby, a pH-sensitive liposome can be obtained that is no longer able to stably retain the membrane structure due to the low pH environment at the tumor site and hence capable of collapsing and releasing the bioactive substance included therein. By using the above-described polyamino acid, the release speed of the bioactive substance can be adjusted.

Furthermore, a polyamino acid comprising a Lys residue, an Arg residue, a Cys residue, an Asp residue, a Glu residue, or a Ser residue has an amino group, a thiol group, a carboxyl group, or a hydroxyl group in the side chain thereof. For this reason, these polyamino acids are capable of reacting with a terminally modified polyoxyalkylene. Because it is thereby possible to confer a polyoxyalkylene chain not only to the termini (N terminus and C terminus) of the polyamino acid, but also to the side chain thereof, it becomes possible to introduce a plurality of polyoxyalkylene chains to the lipid derivative of the present invention. Therefore, when such a lipid derivative is prepared as a liposome, a liposome having two or more polyoxyalkylene chains has better stability and the like, compared to a liposome having a single polyoxyalkylene chain, and in addition, upon cleavage of the polyamino acid chain by an enzyme, the plurality of polyoxyalkylene chains simultaneously leave the liposome surface, so that the release of the bioactive substance can be further promoted.

Each of (OA)ₙ₁, (OA)ₙ₂, and (OA)ₙ₃ independently represents a polyoxyalkylene group whose constituent unit is an oxyalkylene group having 2 to 4 carbon atoms. The oxyalkylene group may be linear or branched; when the polyoxyalkylene group has a plurality of kinds of oxyalkylene groups, the polyoxyalkylene group may be in a block state or random state.

Each of m1, m2, and m3 independently represents a positive number of 0 to 5 that satisfies the requirement of ml+m2+m3=1 to 9. Regarding m1, m2, and m3, from the viewpoint of the stability of the liposome and the promotion of the release of the bioactive substance, the value m1+m2+m3 is preferably 1 to 6, more preferably 2 to 6. The sum of m1, m2 and m3 indicates the total number of polyoxyalkylene chains in the lipid derivative.

Each of n1, n2 and n3 means the average molar number of alkylene oxides added. It is desirable that each of n1, n2 and n3 is independently a positive number of 4 to 800, preferably 6 to 227, that satisfies the requirement of (n1Xm1)+(n2Xm2)+(n3Xm3) = 4 to 2,000, preferably 8 to 1,000, more preferably 22 to 1000, still more preferably 40 to 1000, particularly preferably 40 to 500. When n1, n2 and n3 are smaller than 4, the hydrated layer for conferring stabilization to the lipid membrane structure is not sufficiently retained, so that the stability becomes insufficient. When the upper limit value in the above-described numerical formula is larger than 2,000, the viscosity is high, the workability would be poor, and the handling would be difficult when the lipid derivative is prepared as a solution. Furthermore, the balance between the hydrophilic moiety of the polyoxyethylene chain and the hydrophobic moiety of the lipid in the lipid derivative molecule would be poor, so that the polyoxyalkylene chain-containing lipid derivative would possibly drop off from the membrane structure when forming a lipid membrane structure.

Z is a hydrogen atom or a methyl group and functions as the terminal group of a polyoxyalkylene group.

X¹ is a divalent group comprising -NHCO- or -OOC-. As such, X¹ is, for example, a divalent group formed by a reaction of the C-terminal carboxyl group of the polyamino acid or, when the polyamino acid comprises a glutamic acid residue or an aspartic acid residue, the side chain carboxyl group thereof, with a polyoxyalkylene compound having a terminal amino group or hydroxyl group. X¹ may comprise a linear or cyclic hydrocarbon group, as long as it comprises the above-described binding group. As X¹, groups represented by the following formulas (b1) to (b3) are suitable.

-OCH₂CH₂CH₂NHCO- (b1),

-OCH₂CH₂NHCO- (b2),

-OOC- (b3)

X² is a divalent group comprising a group selected from the group consisting of -OCONH-, -CONH-, -CH₂NH-, and - NHC(O)NH-. As such, X² is, for example, a divalent group formed by a reaction of the N-terminal α-amino group of a polyamino acid or, when the polyamino acid comprises a lysine residue or an arginine residue, the side chain ε-amino group thereof, with a polyoxyalkylene compound having a carbonate group, a carboxyl group or an active ester group thereof, an isocyanate group, or an aldehyde group at the terminus thereof. X² may comprise a linear or cyclic hydrocarbon group, as long as it comprises the above-described binding group. As X², groups represented by the following formulas (c1) to (c7) are suitable.

-OCONH- (c1),

-OCOCH₂CH₂CONH- (c2),

-OCOCH₂CH₂CH₂CONH- (c3),

-OCH₂CONH- (c4),

-O(CH₂)₅CONH- (c5),

-OCONH(CH₂)₆NHCONH- (c6),

-OCH₂CH₂CH₂NH- (c7)

X³ is a divalent group comprising a group selected from the group consisting of >CH-S-, -NHCOO-, -COO-, -COS-, and -O-. As examples of such X³, a divalent group formed by a reaction of the thiol group when the polyamino acid comprises a cysteine residue or the hydroxyl group when the polyamino acid comprises a serine residue, with a polyoxyalkylene compound comprising a terminal maleimide group, or a divalent group formed by a reaction with a polyoxyalkylene compound comprising an isocyanate group, a methanesulfonic acid group, a trifluoroethanesulfonic acid group, a p-toluenesulfonic acid group, a carboxyl group or a glycidyl group can be mentioned. As X³, groups represented by the following formulas (d1) to (d4) are suitable. In the following formulas, a is 2 or 3, and b is 2 to 5.

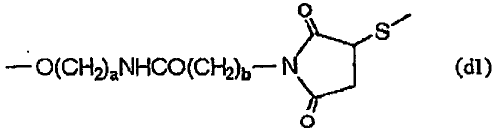

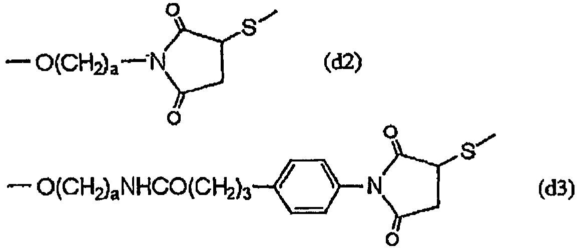

-O- (d4)

The invention also provides a polyoxyalkylene chain-containing lipid derivative as defined above.

Next, methods of producing the polyoxyalkylene chain-containing lipid derivative of the present invention are described.

For example, the lipid derivative can be produced by the following methods.
(i) Method comprising reacting a polyamino acid with a polyoxyalkylene compound, then further reacting with a lipid.
(ii) Method comprising reacting a polyamino acid with a lipid, then further reacting with a polyoxyalkylene compound.

Although any of the above-described methods (i) and (ii) may be adopted, usually, the method (i) is often more reactive than the method (ii). After an amino acid and a polyoxyalkylene compound are reacted, another amino acid is further bound by a peptide linkage to synthesize a polyamino acid, which may then be reacted with a lipid. The reaction of a polyamino acid with a polyoxyalkylene compound can be carried out by, for example, the methods described in the Processes 1 to 6 shown below. The reaction of each of the polyoxyalkylene-polyamino acid compounds obtained by Processes 1 to 6 and a lipid can be carried out by the methods described in the Processes 7 to 10 shown below. The amino group and carboxyl group of a polyamino acid can be used after being protected with a protecting group as described in "Peptide Gosei-no-Kiso to Jikken" (written by Nobuo Izumiya, published by Maruzen Ltd., January 20, 1985) in the synthesis step. (Process 1: a case wherein X¹ is a divalent group comprising - NHCO-)

As the polyoxyalkylene compound, for example, a polyoxyalkylene amine compound wherein the terminus of the polyoxyalkylene group represented by (OA)ₙ₁, (OA)ₙ₂, or (OA)ₙ₃ is an amino group represented by the following formula (e1) or (e2) can be used.

-OCH₂CH₂CH₂NH₂ (e1),

-OCH₂CH₂NH₂ (e2)

As the polyamino acid, for example, a polyamino acid having a C-terminal carboxyl group or a polyamino acid comprising a glutamic acid residue or an aspartic acid residue, having a side chain carboxyl group, can be used. The carboxyl group of the polyamino acid may be used as an activated ester using a dehydrocondensing agent and an activator, or may be activated using a water-soluble carbodiimide such as 1-ethyl-3-(3-dimethylaminopropyl)carbodiimide.

As the dehydrocondensing agent, any one can be used , as long as it is capable of condensing the carboxyl groups of the polyamino acid with dehydration. As the dehydrocondensing agent, carbodiimide derivatives such as dicyclohexylcarbodiimide can be mentioned, and dicyclohexylcarbodiimide is particularly preferable. The amount of dehydrocondensing agent used is 1.05 to 5 equivalents, preferably 1.5 to 2.5 equivalents, relative to the polyoxyalkylene carboxylic acid compound.

An activated ester can be obtained by, for example, reacting a polyamino acid with an activator in the presence of a dehydrocondensing agent. The choice of activator is not particularly limited; for example, N-hydroxysuccinimide, imidecarbonate N,N'-disuccinate, 1-hydroxybenzotriazole, N-hydroxy-5-norbornene-2,3-dicarboxyimide, N-hydroxyphthalimide, 4-hydroxyphenyldimethylsulfonium methyl sulfate, and isobutyl chloroformate can be mentioned. Out of them, N-hydroxysuccinimide is preferable. The amount of N-hydroxysuccinimide used is 0.1 to 2 equivalents relative to the polyamino acid. Thereby, the yield can be increased in some cases.

By reacting a polyoxyalkylene amine compound with a polyamino acid in the presence of a basic catalyst in an organic solvent, a polyoxyalkylene-polyamino acid compound having the above-described divalent group can be produced in high purity. Although the amount of polyoxyalkylene amine compound used is not particularly limited, the equivalent ratio (A:B) of the polyoxyalkylene amine compound (A) to the polyamino acid (B) is preferably 1:1 to 1:5.

The choice of basic catalyst used in the reaction is not particularly limited; for example, a nitrogen-containing substance such as triethylamine or ammonium acetate, and a sodium salt such as sodium phosphate, sodium carbonate, sodium hydrogen carbonate, sodium borate, or sodium acetate can be mentioned. The amount of basic catalyst used is, for example, 1 to 10 equivalents, preferably 1.2 to 5 equivalents, relative to the polyamino acid. Reaction temperature is normally 10 to 90°C, preferably 15 to 50°C, more preferably 20 to 45°C. At temperatures lower than 10°C, the reaction rate is low in some cases; at temperatures higher than 90°C, side-reaction products are formed in some cases. Reaction time is 1 hour or more, preferably 2 to 8 hours.

When an organic solvent having a hydroxyl group, such as ethanol, is used as the organic solvent for the reaction, the hydroxyl group reacts with the terminal carboxyl group of the polyamino acid in some cases. For this reason, as the organic solvent, one not having a reactive functional group such as a hydroxyl group can be used. As examples of such an organic solvent, acetonitrile, dimethylformamide, dimethylsulfoxide, or a mixed solvent thereof and ethyl acetate, dichloromethane, chloroform, benzene, and toluene can be mentioned. Out of them, because of the ease of dissolution of the polyamino acid, acetonitrile and dimethylformamide are preferable.

After completion of the reaction, the product can be purified by performing the steps described below. By a method comprising filtering off insoluble matter from the reaction solution, then concentrating the filtrate or pouring the filtrate in a poor solvent to cause crystallization and the like, a crystal of a polyoxyalkylene-polyamino acid compound can be obtained in high purity. The obtained crystal is dissolved, the solution is cooled or a poor solvent is added to crystallize the polyoxyalkylene-polyamino acid compound, whereby the free polyamino acid, dehydrocondensing agent, N-hydroxysuccinimide, and dicyclohexylcarbodiimide can be removed to purify the crystal. As the solvent used in this step, a solvent capable of dissolving the obtained crystal and crystallyzing the polyoxyalkylene-polyamino acid compound upon cooling, or by the addition of a poor solvent is preferable.

It is desirable that impurities such as salts are removed by a method comprising dissolving the obtained crystal in a solvent such as ethyl acetate, and then adding an adsorbent and performing stirring. As the adsorbent, an alkaline earth metal oxide (e.g., magnesium oxide), an alkaline earth metal hydroxide (e.g., magnesium hydroxide), or an adsorbent comprising aluminum or silicon (e.g., aluminum oxide, aluminum hydroxide, silicon oxide), and activated charcoal can be mentioned. These adsorbents are commercially available; for example, Kyoward 200, Kyoward 300, Kyoward 500, Kyoward 600, Kyoward 700, Kyoward 1000, Kyoward_2000_ (all manufactured by Kyowa Chemical Industry Co., Ltd., trade mark), Tomix-AD300, Tomix-AD500, and Tomix-AD700 (all manufactured by Tomita Pharmaceutical Co., Ltd., trade mark) can be mentioned. Adsorbents can be used singly or in combination of 2 kinds or more.

The temperature for treatment using an adsorbent is 10 to 85°C, preferably 40 to 70°C; treatment time is 10 minutes to 5 hours, preferably 30 minutes to 3 hours. When the temperature for treatment is lower than 10°C, the polyoxyalkylene-polyamino acid compound is crystallized and, during removal of the adsorbent, the polyoxyalkylene-polyamino acid compound is also removed so that the yield tends to decrease. When the temperature exceeds 85°C, hydrolysis of the polyoxyalkylene-polyamino acid compound possibly occur due to the presence of a trace amount of water during the adsorbent treatment. The amount of adsorbent used is 0.1 to 200 parts by weight, preferably 1 to 50 parts by weight, relative to 100 parts by weight of the crystal treated. After the adsorbent treatment, the adsorbent is removed by a method such as filtration, after which the filtrate can be cooled or a poor solvent can be used, to cause crystallization. Preferably, when crystallization is performed with cooling to 10°C or below, a crystal is obtained in a good yield.

The amount of solvent used in the above-described step is 1 to 100 volumes, preferably 2 to 50 volumes, relative to the crystal. After recrystallization, cooling is performed or a poor solvent is used, to cause crystallization. As a specific method of crystallization, the following method can be mentioned. After dissolving in a solvent such as ethyl acetate, toluene, or chloroform, ether or an aliphatic hydrocarbon solvent having 5 to 8 carbon atoms is added, whereby a crystal of the polyoxyalkylene-polyamino acid compound is precipitated. Specifically, a method comprising dissolving using ethyl acetate, and then adding hexane to cause crystallization, is preferable. The aliphatic hydrocarbon having 5 to 8 carbon atoms is not particularly limited; for example, pentane, isopentane, neopentane, hexane, isohexane, 3-methypentane, neohexane, 2,3-dimethylbutane, heptane, 2-methyhexane, 3-methyhexane, 3-ethylpentane, 2,2-dimethylpentane, 2,3-dimethylpentane, 3,3-dimethylpentane, 2,3,3-trimethybutane, octane, 2-methylheptane, 3-methylheptane, 4-methylheptane, 3-ethylhexane, 2,2-dimethylhexane, 2,3-dimethylhexane, 2,4-dimethylhexane, 2,5-dimethylhexane, 3,3-dimethylhexane, 3,4-dimethylhexane, 2-methyl-3-ethylpentane, 3-methyl-3-ethylpentane, 2,2,3-trimethylpentane, 2,2,4-trimethylpentane, and 2,2,3,3-tetramethylbutane can be mentioned. Out of them, hexane and heptane are preferable. When a further improvement in crystal purity is desired, a polyoxyalkylene-polyamino acid compound of still higher purity can be obtained by repeating the same crystallization step several times.

### (Process 2: a case wherein X² is a divalent group comprising - CONH-)

As the polyoxyalkylene compound, for example, a polyoxyalkylene carboxylic acid compound wherein the above-described terminus of polyoxyalkylene group is a carboxylic acid represented by one of the following formulas (f1) to (f4) can be used.

-OCOCH₂CH₂COOH (f1),

-OCOCH₂CH₂CH₂COOH (f2),

-OCH₂COOH (f3),

-O(CH₂)₅COOH (f4)

These terminal carboxyl groups may be converted to acid anhydrides using a dehydrocondensing agent, and may be converted to activated esters using an activator. As the dehydrocondensing agent and the activator, the same compounds as those mentioned for the Process 1 above can be mentioned.

As examples of the polyamino acid, a polyamino acid having an N-terminal α-amino group or a polyamino acid comprising a lysine residue having an ε-amino group can be mentioned. By reacting the above-described polyoxyalkylene carboxylic acid compound with polyamino acid in the presence of a basic catalyst in an organic solvent, a polyoxyalkylene-polyamino acid compound having the above-described divalent group can be produced in high purity. Although the amount of polyoxyalkylene carboxylic acid compound used is not particularly limited, the equivalent ratio (C:B) of the polyoxyalkylene carboxylic acid compound (C) to the polyamino acid (B) is preferably 1:1 to 1:5. Other reaction conditions, and purification step are the same as those for the Process 1 above.

### (Process 3: a case wherein X² is a divalent group comprising - OCONH-)

As the polyoxyalkylene compound, for example, a polyoxyalkylene carbonate compound wherein the above-described terminus of polyoxyalkylene group is carbonated can be used. As examples of the polyoxyalkylene carbonate compound, a polyoxyalkylene-p-nitrophenyl carbonate can be mentioned. As the polyamino acid, for example, a polyamino acid having an N-terminal α-amino group or a polyamino acid comprising a lysine residue having an ε-amino group can be used. By reacting the polyoxyalkylene carbonate compound with polyamino acid in the presence of a basic catalyst in an organic solvent, a polyoxyalkylene-polyamino acid compound having the above-described divalent group can be produced in high purity. Although the amount of polyoxyalkylene carbonate compound used is not particularly limited, the equivalent ratio (D:B) of the polyoxyalkylene carbonate compound (D) to the polyamino acid (B) is preferably 1:1 to 1:5. With respect to reaction conditions and purification step, this reaction can be carried out by the same method as the Process 1 above.

### (Process 4: a case wherein X² is a divalent group comprising - CH₂NH-)

As the polyoxyalkylene compound, for example, a polyoxyalkylene aldehyde compound wherein the above-described terminus of polyoxyalkylene group is an aldehyde group can be used. As the polyoxyalkylene aldehyde compound, for example, a polyoxyalkylene aldehyde compound having an aldehyde group represented by the following formula (g1) can be used. In the following formula, P is an alkylene group such as a methylene group, an ethylene group, a trimethylene group, or a tetramethylene group.

-O-P-CHO (g1)

As the polyamino acid, for example, a polyamino acid having an N-terminal α-amino group or a polyamino acid comprising a lysine residue having an ε-amino group can be used. By reacting the polyoxyalkylene aldehyde compound with polyamino acid in the presence of a reducing agent in a buffer solution, a polyoxyalkylene-polyamino acid compound having the above-described divalent group can be produced in high purity. Although the amount of polyoxyalkylene aldehyde compound used is not particularly limited, the equivalent ratio (E:B) of the polyoxyalkylene aldehyde compound (E) to the polyamino acid (B) is preferably 1:1 to 1:5.

As the above-described buffer solution, an acetate buffer solution, a phosphate buffer solution, and a tris-acid buffer solution are suitably used. An organic solvent not involved in the reaction, such as acetonitrile, dimethylsulfoxide, dimethylformamide, or dimethylacetamide, may be further added. The pH during the reaction is pH 2 to 8.5, preferably pH 3 to 7. Reaction temperature is 0 to 90°C, and reaction time is 0.5 to 20 hours, preferably 0.5 to 4 hours. In the absence of the above-described reducing agent, a Schiff base is formed. When a Schiff base is formed, this is subjected to reducing treatment using a reducing agent such as sodium borocyanohydride to form a secondary amino group. After the reaction, the product can be purified using means of purification such as dialysis, salting-out, ultrafiltration, ion exchange chromatography, electrophoresis, extraction, recrystallization, adsorption treatment, re-precipitation, and column chromatography.

### (Process 5: a case wherein X¹ is a divalent group comprising - OOC-)

As the polyoxyalkylene compound, for example, a polyoxyalkylene compound wherein the above-described terminus of polyoxyalkylene group is a hydroxyl group can be used. As the polyamino acid, for example, a polyamino acid having a C-terminal carboxyl group or a polyamino-acid comprising a glutamic acid residue or aspartic acid residue, having a side chain carboxyl group can be used. The carboxyl group of the polyamino acid may be used as an activated ester by the same method as the Process 2 above, or may be activated using a water-soluble carbodiimide. Reaction conditions and purification conditions are the same as those for the Process 2 above.

### (Process 6: a case wherein X³ is a divalent group comprising >CH-S-)

As the polyoxyalkylene compound, for example, a polyoxyalkylene maleimide compound wherein the above-described terminus of polyoxyalkylene group is a maleimide group can be used. As the polyoxyalkylene maleimide compound, a polyoxyalkylene maleimide compound having a maleimide group represented by one of the following formulas (h1) to (h3) can be mentioned. In the following formulas, a is 2 or 3, and b is 2 to 5.

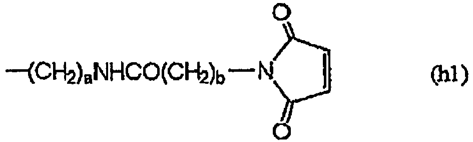

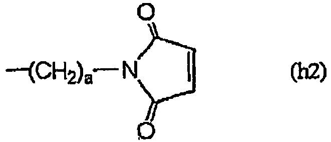

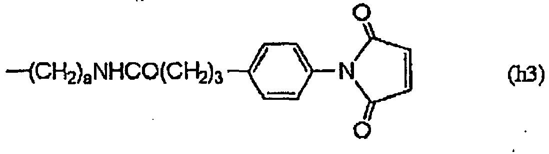

As the polyamino acid, for example, a polyamino acid having a thiol group derived from a serine residue or a polyamino acid comprising an amino acid residue incorporating a thiol group introduced using iminothiolane can be used. By reacting the above-described polyoxyalkylene maleimide compound with a polyamino acid in a buffer solution, a sulfide bond is formed. As the above-described buffer solution, buffer solutions such as a phosphate buffer solution, a borate buffer solution, a tris-acid buffer solution, and an acetate buffer solution are preferable. As in the Process 4, an organic solvent may be added. Reaction temperature is not particularly limited, and is preferably 0 to 80°C. Reaction time is preferably 0.5 to 72 hours, more preferably 1 to 24 hours.

### (Process 7: a case wherein Y is a divalent group comprising - CONH-)

As the lipid, a phospholipid comprising an amino group, such as phosphatidylethanolamine, can be used. By reacting the phosphatidylethanolamine with a polyoxyalkylene-polyamino acid compound comprising a carboxyl group, obtained by the above-described method, in the presence of a basic catalyst in an organic solvent, a polyoxyalkylene chain-containing lipid derivative having the above-described divalent group, can be produced. This reaction is normally carried out using a dehydrocondensing agent. The choice of basic catalyst is not particularly limited; for example, nitrogen-containing substances such as triethylamine, pyridine, dimethylaminopyridine, and ammonium acetate, and sodium salts such as sodium phosphate, sodium carbonate, sodium hydrogen carbonate, sodium borate, and sodium acetate can be mentioned. The amount of basic catalyst used is, for example, 1.5 to 10 equivalents, preferably 2 to 5 equivalents, relative to the polyoxyalkylene-polyamino acid compound.

When an organic solvent having a hydroxyl group, such as ethanol, is used as the organic solvent, it reacts with the carboxyl group of the polyoxyalkylene-polyamino acid compound in some cases. For this reason, as the organic solvent, any one not having a reactive functional group such as a hydroxyl group can be used. As such an organic solvent, for example, ethyl acetate, dichloromethane, chloroform, benzene, toluene, or a mixed solvent thereof can be used. Out of them, chloroform and toluene are preferable. For some kinds of polyoxyalkylene-polyamino acid compounds, the reaction can be carried out even in an aqueous solution such as a buffer solution. In this case, as the water-soluble dehydrocondensing agent, 1-ethyl-3-(3-dimethylaminopropyl) carbodiimide may be used. Even in the reaction in an organic solvent, a dehydrocondensing agent can be used. The dehydrocondensing agent is not particularly limited, as long as it is capable of condensing the carboxyl group of the polyoxyalkylene-polyamino acid compound and the amino group of the phospholipid with dehydration. As such a dehydrocondensing agent, a carbodiimide compound such as dicyclohexylcarbodiimide can be mentioned, and dicyclohexylcarbodiimide is particularly preferable. The amount of dehydrocondensing agent used is, for example, 1.05 to 5 equivalents, preferably 1.5 to 2.5 equivalents, relative to the polyoxyalkylene-polyamino acid compound. By adding an activator to the reaction system at 0.1 to 2 equivalents relative to the palyoxyalkylene-polyamino acid compound, the lipid derivative can also be reacted as an activated ester form. The choice of activator is not particularly limited; for example, N-hydroxysuccinimide, imidecarbonate N,N'-disuccinate, 1-hydroxybenzotriazole, N-hydroxy-5-norbornene-2,3-dicarboxyimide, N-hydroxyphthalimide, 4-hydroxyphenyldimethylsulfonium methyl sulfate, and isobutyl chloroformate can be used.

Reaction temperature is normally in the range of 20 to 90°C, preferably 40 to 80°C. Reaction time is 1 hour or more, preferably 2 to 8 hours. At temperatures lower than 20°C, the reaction rate is low; at temperatures higher than 90°C, the acyl group of the phospholipid used in the reaction undergoes hydrolysis in some cases.

### (Process 8: a case wherein Y is a divalent group comprising - NHCO-)

By reacting a phospholipid comprising a carboxyl group with the polyoxyalkylene-polyamino acid compound comprising an amino group, obtained by the above-described method, in the presence of a basic catalyst in an organic solvent, a polyoxyalkylene chain-containing lipid derivative having the above-described divalent group, can be produced. This reaction is normally carried out using a dehydrocondensing agent. The phospholipid comprising a carboxyl group can easily be produced by reacting a phospholipid with a dicarboxylic anhydride. As examples of the dicarboxylic anhydride, anhydrides of oxalic acid, malonic acid, succinic acid, glutaric acid, adipic acid, pimelic acid, suberic acid, azelaic acid, sebacic acid, maleic acid, fumaric acid, phthalic acid, and terephthalic acid can be mentioned. The dicarboxylic anhydride used may be an intramolecular anhydride or an intermolecular anhydride. Out of them, an anhydride of succinic acid and glutaric acid and an anhydride of succinic acid or glutaric acid are preferable.

The reaction of the phospholipid comprising a carboxyl group with the activator can be carried out in the presence of a dehydrocondensing agent, in a solvent that is not reactive with carboxylic acid (e.g., chloroform, toluene) at a reaction temperature of 15 to 80°C, preferably 25 to 55°C, in the same manner as the reaction of the polyoxyalkylene compound with the dicarboxylic anhydride. In this case, the reaction can be carried out by, for example, dispersing the activator in a solution of the polyoxyalkylene-polyamino acid compound with stirring. As the activator, the same as the above-described activator can be used. The reaction of the phospholipid with the polyoxyalkylene-polyamino acid compound can be carried out under the same conditions as Process 7.

### (Process 9: a case wherein Y is a divalent group comprising >CHS-)

As the lipid, a phospholipid compound having a maleimide group, represented by one of the following formulas (j1) to (j2), at the terminus of phosphatidylethanolamine can be used. In the following formulas, b is 2 to 5.

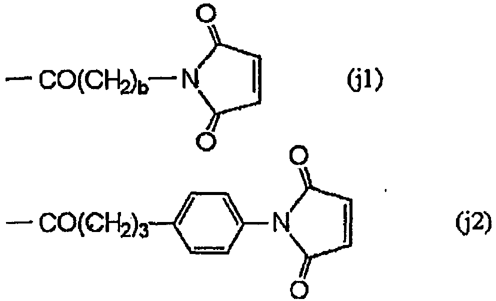

As the polyamino acid, a polyamino acid having a thiol group derived from a serine residue, a polyamino acid comprising an amino acid residue incorporating a thiol group introduced using iminothiolane can be used. By reacting the above-described phospholipid with polyamino acid in a buffer solution, a sulfide bond is formed. As the above-described buffer solution, a phosphate buffer solution, a borate buffer solution, and a tris-acid buffer solution, an acetate buffer solution are suitable. In the above-described reaction, an organic solvent may be added as in Process 4. Reaction temperature is not particularly limited, and is preferably 0 to 80°C. Reaction time is preferably 0.5 to 72 hours, more preferably 1 to 24 hours.

### (Process 10: a case wherein Y is a divalent group comprising - OCONH-)

As the lipid, for example, a terminally carbonated cholesterol carbonate compound can be used. As examples of the cholesterol carbonate compound, cholesteryl-p-nitrophenylcarbonate can be mentioned. As the polyamino acid, for example, a polyamino acid having an N-terminal α-amino group or a polyamino acid comprising a lysine residue having an ε-amino group can be used.

By reacting the above-described cholesterol carbonate compound with a polyamino acid in the presence of a basic catalyst in an organic solvent, a cholesterol carbonate-polyamino acid compound can be produced in high purity. Although the amount of cholesterol carbonate compound used is not particularly limited, the equivalent ratio (F:B) of the cholesterol carbonate compound (F) to the polyamino acid (B) is preferably 1:1 to 1:5. Reaction conditions and purification step are the same as those for the Process 7 above.

The average molecular weight of the polyoxyalkylene chain-containing lipid derivative obtained by the above-described production method, is preferably 1000 to 90000, more preferably 1200 to 90000, still more preferably 2500 to 90000. When the molecular weight is lower than 1000, the hydrated layer for conferring stabilization to the lipid membrane structure is not sufficiently retained, so that the stability becomes insufficient. When the molecular weight is higher than 90000, the workability becomes poor and the handling becomes difficult when the lipid derivative is prepared into a solution. Moreover, when a lipid membrane structure is formed, the polyoxyalkylene chain-containing lipid derivative possibly drops off from the membrane structure.

Next, the lipid membrane structure of the present invention is described. The lipid membrane structure of the present invention comprises a polyoxyalkylene chain-containing lipid derivative represented by the formula (1) above and a phosphatidylethanolamine as a constituent of the lipid membrane. Here, "a lipid membrane structure" in the present invention means a particle having a membrane structure wherein hydrophilic groups of an amphiphilic lipid are arranged toward the water phase of the interface. The form of the lipid membrane structure is not particularly limited; for example, a form of a dried lipid mixture and a form dispersed in an aqueous solvent, as well as a dried form thereof, and a frozen form thereof can be mentioned. In the case of a form of a dried lipid mixture, a lipid membrane structure can be produced by, for example, once dissolving a lipid ingredient in an organic solvent such as chloroform, and then performing evaporation to dryness under reduced pressure using an evaporator or spray-drying using a spray dryer. As the form dispersed in an aqueous solvent, single-membrane liposome, multilamellar liposome, O/W type emulsion, W/O/W type emulsion, spherical micelles, wormlike micelles, and amorphous and lamella structures can be mentioned. Out of them, liposomes are preferable. For example, in the case of a liposome, a polyoxyalkylene chain-containing lipid derivative is used in a ratio of 0.1 to 25% by weight, preferably 0.1 to 20% by weight, relative to the total weight of the lipid membrane structure, liposome.

The constituent lipid of a lipid membrane structure in the present invention, is phosphatidylethanolamine. As mentioned herein, the constituent of a lipid membrane structure indicates a constituent other than the polyoxyalkylene-chain-containing lipid derivative. Phosphatidylethanolamine, having a saturated or unsaturated, linear or branched acyl group having 4 to 24, preferably 12 to 20, carbon atoms, can be mentioned. A lipid derived from a natural product, such as egg yolk lecithin or soybean lecithin, may be blended.

When an acidic phospholipid, for example, a phospholipid comprising a carboxyl group at the terminus of a hydrophilic group, is used as the constituent lipid of a liposome, because the pH is low, for example, 6 or below, preferably 5 or below, and the carboxyl group is protonized, the charge of the lipid membrane surface is lost so that the membrane becomes no longer stable. That is, the liposome exhibits pH sensitivity. An acidic phospholipid can be produced by, for example, reacting a phospholipid compound with a dicarboxylic anhydride. A phospholipid compound may be a natural phospholipid or a synthetic phospholipid. As examples of a natural phospholipid, a natural phosphatidylethanolamine such as soybean phosphatidylethanolamine or egg yolk phosphatidylethanolamine can be mentioned. As examples of a synthetic phospholipid, a hydrogenated natural phosphatidylethanolamine such as hydrogenated soybean phosphatidylethanolamine and hydrogenated egg yolk phosphatidylethanolamine can be mentioned. Furthermore, when a liposome is prepared using a polyoxyalkylene chain-containing phospholipid derivative wherein the terminus of the phospholipid compound becomes a carboxy group upon hydrolysis of the amino acid moiety of the polyoxyalkylene chain-containing phospholipid derivative, upon cleavage of the polyoxyalkylene chain, the hydrated layer of the liposome surface is lost, and the terminus of the phospholipid compound becomes a carboxyl group. For this reason, as in the above-described case using an acidic phospholipid, the liposome exhibits pH sensitivity and an equivalent effect is obtained.

When a phospholipid having an acyl group derived from oleic acid, particularly dioleoylphosphatidylethanolamine, is used as the constituent lipid of the liposome, it is desirable to use a polyoxyalkylene chain-containing lipid derivative produced using dioleoylphosphatidylethanolamine as the base material compound. When the major constituent of the liposome is dioleoylphosphatidylethanolamine, the dioleoylphosphatidylethanolamine becomes likely to form a hexagonal structure or a reverse-micelle structure at low pH levels, for example, pH 6 or below, preferably pH 5 or below. For this reason, upon cleavage of the polyoxyalkylene chain covering the liposome surface, the hydrated layer of the liposome surface is lost, so that pH sensitivity, a property of liposome, is exhibited. Thereby, a bioactive substance and the like encapsulated in the liposome can be efficiently released at a target site such as a tumor site.

The size of the lipid membrane structure in the form of dispersion in an aqueous solvent is not particularly limited; for example, in the case of a liposome or an emulsion, the particle diameter is 50 nm to 5 µm; in the case of spherical micelles, the particle diameter is 5 nm to 100 nm. In the case of wormlike micelles and amorphous and lamella structures, the thickness per layer is 5 nm to 10 nm, and a plurality of such layers are formed. In the present invention, particle diameter refers to one measured by the dynamic light scattering method.

The choice of aqueous solvent (dispersion medium) is not particularly limited; for example, buffer solutions such as a phosphate buffer solution, a citrate buffer solution, and phosphate buffered physiological saline, physiological saline, and culture medium for cell culture can be used. When the polyoxyalkylene chain-containing lipid derivative of the present invention is used in dispersion in an aqueous solvent, a stably dispersed lipid membrane structure can be obtained, and in addition to water, an aqueous solution of a sugar such as glucose, lactose, or sucrose, a polyhydric alcohol such as glycerin, and propylene glycol may be added. To preserve the lipid membrane structure in dispersion in an aqueous solvent stably for a long time, it is desirable, from the viewpoint of physical stability properties such as aggregation, that the electrolytes in the aqueous solvent are decreased to the maximum possible extent, and it is also desirable to remove dissolved oxygen by nitrogen bubbling. Furthermore, when the lipid membrane structure is preserved under freeze-drying or spray-drying, for example, when a lipid membrane structure in dispersion in an aqueous solvent is preserved under freezing, long-term preservation becomes possible by using an aqueous solution of a sugar or an aqueous solution of a polyhydric alcohol. The concentration of the aqueous solvent is not particularly limited; for example, in an aqueous solution of a sugar, the concentration is preferably 2 to 20% (W/V), more preferably 5 to 10% (W/V). In an aqueous solution of a polyhydric alcohol, the concentration is preferably 1 to 5% (W/V), more preferably 2 to 2.5% (W/V). In a buffer solution, the buffering agent concentration is preferably 5 to 50 mM, more preferably 10 to 20 mM. Although the concentration of the lipid membrane structure in the aqueous solvent is not particularly limited, the total concentration of the lipid in the aqueous solvent is preferably 0.1 to 500 mM, more preferably 1 to 100 mM.

A lipid membrane structure in the form of a dispersion in an aqueous solvent can be produced by adding the above-described dried lipid mixture to the aqueous solvent, and emulsifying the mixture using an emulsifying machine such as a homogenizer, a sonication emulsifying machine, and a high-pressure spray emulsifying machine. The method of producing the liposome is not particularly limited; the liposome can also be produced by a well known method, for example, reversed phase evaporation. When the size of the lipid membrane structure is to be controlled, extrusion (extrusion filtration) may be performed under high pressure using a membrane filter with uniform pore diameter.

As the method of further drying the lipid membrane structure in dispersion in the aqueous solvent, ordinary freeze-drying and spray-drying can be mentioned. In this case, as the aqueous solvent, an aqueous solution of a sugar, preferably an aqueous solution of sucrose or an aqueous solution of lactose can be used as described above. When the lipid membrane structure dispersed in an aqueous solvent is further dried after production, long-term preservation of the lipid membrane structure becomes possible. By adding an aqueous solution of a pharmaceutical to the dried lipid membrane structure, the lipid mixture is efficiently hydrated, so that the pharmaceutical can be efficiently retained in the lipid membrane structure.

As the bioactive substance that can be retained in the lipid membrane structure, an ingredient that regulates the function in vivo can be mentioned; the choice thereof is not particularly limited. As the bioactive substance, a vitamin, a neurotransmitter, a protein, a polypeptide, a drug, and a gene can be mentioned. As examples of the vitamin, vitamin A, vitamin B, vitamin C, vitamin D, vitamin E, and vitamin K can be mentioned. As examples of the protein, the polypeptide, hormones, serum proteins, immunoglobulins, interleukins, interferons (-α, -β, -γ), granulocyte colony stimulating factor (α and p types), macrophage colony stimulating factor, granulocyte macrophage colony stimulating factor, platelet-derived growth factor, phospholipase activated protein, insulin, and monoclonal/polyclonal antibodies and fragments thereof can be mentioned. As the drug, an anticancer agent, and an antifungal agent can be mentioned. As examples of the anticancer agent, paclitaxel, adriamycin, doxorubicin, cisplatin, daunomycin, mitomycin, vincristine, epirubicin, methotrexate, and 5-fluorouracil can be mentioned. The choice of antifungal agent is not particularly limited; for example, amphotericin B, nystatin, flucytosine, miconazole, fluconazole, itraconazole, ketoconazole and a peptide-based antifungal agent can be mentioned. As examples of the gene, oligonucleotide, DNA, and RNA can be mentioned.

### [Examples]

The present invention is hereinafter described in more detail by means of the following Examples.

### (Example 1)

### Synthesis of methylpolyoxyethylenecarbamyl-glycyl-glycyl-glycyl-dioleoylphosphatidylethanolamine (DOPE-GGG-PEG2000)

### (1) Synthesis of methylpolyoxyethylenecarbamyl-glycyl-glycyl-glycine

5 g (2.5 mmol) of methoxypolyethylene glycol-p-nitrophenyl carbonate (trade name: SUNBRIGHT MENP-20H, weight-average molecular weight 2,000, manufactured by NOF Corporation) was dissolved in 15 mL of acetonitrile. An aqueous solution of 946 mg of glycyl-glycyl-glycine dissolved in 10 mL of water was added to this solution, the mixture was stirred, 0.5 g of triethylamine was added, and the mixture was stirred at room temperature for 3 hours. After completion of the reaction, insoluble matter was removed by filtration, subsequently the solvent was removed under reduced pressure using an evaporator. Next, 50 mL of ethyl acetate was added to dissolve the residue, sodium sulfate was added, and the mixture was stirred, after which the mixture was filtered and dehydrated. Next, 100 mL of hexane was added to the filtrate, and the mixture was cooled to 0°C or below, after which the mixture was filtered to give a crude crystal. The crude crystal was dissolved in 50 mL of ethyl acetate, Kyoward #2000 (0.1 g) and Kyoward #700 (1 g) were added as the adsorbents, and the solution was stirred at 60°C for 1 hour. After the adsorbents were filtered off, 100 mL of hexane was added, and the mixture was cooled to cause crystallization. After the crystal was collected by filtration, the same crystallization as described above was performed once again, and the crystal was dried to give 3 g of the desired compound (yield 52.2%).

The progression of the reaction and identification of the product were performed by thin-layer chromatography (TLC) using a silica gel plate. As the developing solvent, a mixed solvent of chloroform and methanol in a mixing ratio (ratio by volume) of 85:15 was used; a color was developed with iodine vapor, and the Rf value was compared with that of a standard substance to qualitatively determine the substance. The reaction end point was confirmed by the fact that the spot of methoxypolyethylene glycol-p-nitrophenyl carbonate detected near an Rf value of 0.6 and the spot of glycyl-glycyl-glycine detected near an Rf value of 0.1 were converted to a spot detected near an Rf of 0.35 by the above-described TLC. Regarding the identification of the product, the presence of the monomethoxyoxyethylene chain and peptide chain was confirmed by the fact that ¹H-NMR (400 MHz, CDCl₃) detected the methyl group of the terminal methoxy derived from the methoxypolyethylene glycol near δ: 3.3 ppm, the ethylene group of the ethylene glycol near δ: 3.5 ppm, and the methylene group derived from the peptide near δ: 1.5 ppm, respectively.

### (2) Synthesis of methylpolyoxyethylenecarbamyl-glycyl-glycyl-glycyl-dioleoylphosphatidylethanolamine

2 g (0.90 mmol) of the methylpolyoxyethylenecarbamyl-glycyl-glycyl-glycine obtained in (1) above was added to 10 mL of chloroform, and the mixture was dissolved with stirring at 40 to 45°C, after which 1.34 g (1.80 mmol) of dioleoylphosphatidylethanolamine was added, and the mixture was stirred. Next, 1.86 g (9.03 mol) of dicyclohexylcarbodiimide was added, and the mixture was stirred, after which 27 mg (0.27 mmol) of triethylamine was added, and they were reacted for 4 hours. After completion of the reaction, insoluble matter was removed by filtration, subsequently the filtrate was evaporated to remove the chloroform using an evaporator, the residue was re-dissolved in 2 mL of chloroform, and column separation and purification were performed under the conditions shown below. 150 g of WakogelC-100 was used as the stationary phase, and a mixed solvent of chloroform and methanol was used as the mobile phase. Fractionation was performed while the mixing ratio (ratio by volume) of chloroform/methanol was changed from 95/5 to 85/15. The obtained fraction was treated to remove the solvent, 10 mL of hexane was added to cause crystallization, the crystal was collected by filtration and dried to give 400 mg of the title compound.

The progression of the reaction and identification of the product were performed by the same thin-layer chromatography (TLC) as described above except that a mixed solvent of chloroform, methanol and water in a mixing ratio (ratio by volume) of 65:25:4 was used as the developing solvent. The progression of the reaction was confirmed by the fact that the spot of methylpolyoxyethylenecarbamyl-glycyl-glycyl-glycine detected near an Rf value of 0.5 and the spot of dioleoylphosphatidylethanolamine detected near an Rf value of 0.6 were converted to a spot detected near an Rf of 0.75. Regarding the identification of the product, the presence of the monomethoxyoxyethylene chain, peptide chain and dioleoylphosphatidylethanolamine was confirmed by the fact that ¹H-NMR (400 MHz, CDCl₃) detected the methyl group of the terminal methoxy derived from the methoxypolyethylene glycol near δ: 3.4 ppm, the polyoxyethylene group near δ: 3.5 ppm, the methylene group derived from the peptide near δ: 1.5 ppm, and the terminal methyl group of the acyl group derived from the dioleoylphosphatidylethanolamine near δ: 0.9 ppm, respectively. By MALDI-TOF/MS (BIFLEX III: manufactured by BRUKER) analysis, the average molecular weight of the obtained compound was determined to be 3100.

### (Example 2)

### Synthesis of methylpolyoxyethylenecarbamyl-glycyl-lysine (methylpolyoxyethylenecarbamyl)-glycyl-glycyl-glycyl-distearoylphosphatidylethanolamine (DSPE-GGG-K(PEG2000)-G-PEG2000)

### (1) Synthesis of methylpolyoxyethylenecarbamyl-glycyl-lysine (methylpolyoxyethylenecarbamyl)-glycyl-glycyl-glycyl

10 g (5 mmol) of methoxypolyethylene glycol-p-nitrophenyl carbonate (SUNBRIGHT MENP-20H, weight-average molecular weight 2000, manufactured by NOF Corporation) was dissolved in 30 mL of acetonitrile, an aqueous solution of 1.6 g of glycyl-lysine-glycyl-glycyl-glycine (SEQ ID NO:11) dissolved in 18 mL of water was added to this solution, the mixture was stirred, 1 g of triethylamine was added, and the mixture was stirred at room temperature for 3 hours. After completion of the reaction, insoluble matter was removed by filtration, and subsequently the solvent was removed under reduced pressure using an evaporator. Next, 50 mL of ethyl acetate was added to dissolve the residue, sodium sulfate was added, and the mixture was stirred, after which the mixture was filtered and dehydrated. Next, 100 mL of hexane was added to the filtrate, and the mixture was cooled to 0°C or below, after which the mixture was filtered to give a crude crystal. The crude crystal was dissolved in 50 mL of ethyl acetate, an adsorbent treatment was performed by the same method as Synthesis Example 1 to cause crystallization, and crystallization was further performed to give 6.2 g of the desired compound (yield 55%).

The progression of the reaction and identification of the product were performed by thin-layer chromatography (TLC) using a silica gel plate. As the developing solvent, a mixed solvent of chloroform and methanol (mixing ratio (ratio by volume) was 85:15) was used; a color was developed with iodine vapor, and the Rf value was compared with that of a standard substance to qualitatively determine the substance. The reaction end point was confirmed by the fact that the spot of methoxypolyethylene glycol-p-nitrophenyl carbonate detected near an Rf value of 0.6 and the spot of glycyl-lysine-glycyl-glycyl-glycine detected near an Rf value of 0.1 were converted to a spot detected near an Rf of 0.35 by the above-described TLC. Regarding the identification of the product, the presence of the monomethoxyoxyethylene chain and peptide chain was confirmed by the fact that ¹H-NMR (400 MHz, CDCl₃) detected the methyl group of the terminal methoxy derived from the methoxypolyethylene glycol near δ: 3.3 ppm, the ethylene group of the ethylene glycol near δ: 3.5 ppm, and the methylene group derived from the peptide near δ: 1.5 ppm, respectively.

### (2) Synthesis of methylpolyoxyethylenecarbamyl-glycyl-lysine (methylpolyoxyethylenecarbamyl)-glycyl-glycyl-glycyl-distearoylphosphatidylethanolamine

4 g (0.90 mmol) of the methylpolyoxyethylenecarbamyl-glycyl-lysine (methylpolyoxyethylenecarbamyl)-glycyl-glycyl-glycyl obtained in (1) above was added to 20 mL of chloroform, and the mixture was dissolved with stirring at 40 to 45°C, after which 1.34 g (1.80 mmol) of distearoylphosphatidylethanolamine was added, and the mixture was stirred. Next, 1.86 g (9.03 mol) of dicyclohexylcarbodiimide was added, and the mixture was stirred, after which 27 mg (0.27 mmol) of triethylamine was added, and they were reacted for 4 hours. After completion of the reaction, insoluble matter was removed by filtration, subsequently the filtrate was treated to remove the chloroform using an evaporator, the residue was re-dissolved in 5 mL of chloroform, and column separation and purification were performed under the conditions shown below. 150 g of WakogelC-100 was used as the stationary phase, and a mixed solvent of chloroform and methanol was used as the mobile phase. Fractionation was performed while the mixing ratio (ratio by volume) of chloroform/methanol was changed from 95/5 to 85/15. The obtained fraction was treated to remove the solvent, 20 mL of hexane was added to cause crystallization, the crystal was collected by filtration and dried to give 700 mg of the title compound.

The progression of the reaction and identification of the product were performed by thin-layer chromatography (TLC) in the same manner as described above except that a mixed solvent of chloroform, methanol and water (mixing ratio (ratio by volume) was 65:25:4) was used as the developing solvent. The reaction end point was confirmed by the fact that the spot of methylpolyoxyethylenecarbamyl-glycyl-lysine (methylpolyoxyethylenecarbamyl)-glycyl-glycyl-glycyl detected near an Rf value of 0.5 and the spot of distearoylphosphatidylethanolamine detected near an Rf value of 0.6 were converted to a spot detected near an Rf of 0.75. Regarding the identification of the product, the presence of the monomethoxyoxyethylene chain, peptide chain and distearoylphosphatidylethanolamine was confirmed by the fact that ¹H-NMR (400 MHz, CDCl₃) detected the methyl group of the terminal methoxy derived from the methoxypolyethylene glycol near δ: 3.4 ppm, the polyoxyethylene group near δ: 3.5 ppm, the methylene group derived from the peptide near δ: 1.5 ppm, and the terminal methyl group of the acyl group derived from the dioleoylphosphatidylethanolamine near δ: 0.9 ppm, respectively. By MALDI-TOF/MS (BIFLEX III: manufactured by BRUKER) analysis, the average molecular weight of the obtained title compound was determined to be 5250.

### (Example 3)

### Synthesis of methylpolyoxyethylenecarbamyl-cysteine (methylpolyoxyethylenemaleimide)-glycyl-glycyl-distearoylphosphatidylethanolamine (DSPE-GGC(-PEG2000)-PEG5000)

### (1) Synthesis of methylpolyoxyethylenecarbamyl-cysteine

150 mL of a 0.1 M, pH 9.5 borate buffer solution was added to 2.5 g of L-cystine, and stirring was performed until the latter dissolved completely. 50 g (10 mmol) of methoxypolyethylene glycol-p-nitrophenyl carbonate (trade name: SUNBRIGHT MENP-50H, average molecular weight 5000, manufactured by NOF Corporation) was dissolved in 50 mL of water, this solution was added to the foregoing L-cystine solution, and while maintaining a pH of 9.5, they were reacted at room temperature for 2 hours. After completion of the reaction, the reaction mixture was adjusted to pH 5.5 with dilute hydrochloric acid, and cooled to 2°C. Next, insoluble matter was removed by filtration, and the filtrate was dialyzed against 2 L of ion-exchange water five times using a dialysis tube. After completion of the dialysis, the solution was adjusted to pH 7.5 and cooled to 2°C, and 2.8 g of 1,4-dithiothreitol was added to cause reduction. After the reduction, dialysis was performed using a 1.5% aqueous solution of acetic acid, and the dialysate was freeze-dried to give 25 g of a crystal of methylpolyoxyethylenecarbamyl-cysteine.

### (2) Synthesis of methylpolyoxyethylenecarbamyl-cysteine (methylpolyoxyethylenemaleimide)

5 g of the methylpolyoxyethylenecarbamyl-cysteine obtained in (1) above was dissolved in 50 mL of physiological saline buffer solution (PBS), 2 g of methoxypolyethylene glycol-propylmaleimide (trade name: SUNBRIGHT ME-020MA, average molecular weight 2,000, manufactured by NOF Corporation) was added, and they were reacted at room temperature for 8 hours. After the reaction, the reaction mixture was adjusted to pH 2, sodium chloride was added, and the mixture was dissolved to obtain a 20%w/w aqueous solution. Next, 50 mL of chloroform was added to cause extraction, after which the extract was treated to remove the solvent using an evaporator, and the residue was re-dissolved in ethyl acetate. Next, hexane was added to cause crystallization, and the crystal was collected by filtration to give 4.5 g of a crystal of methylpolyoxyethylenecarbamyl-cysteine (methylpolyoxyethylenemaleimide).

### (3) Synthesis of methylpolyoxyethylenecarbamyl-cysteine (methylpolyoxyethylenemaleimide)-glycyl-glycyl

4 g of the methylpolyoxyethylenecarbamyl-cysteine (methylpolyoxyethylenemaleimide) obtained in (2) above was dissolved in 80 mL of chloroform, 0.1 g of N-hydroxysuccinimide was added, and the mixture was stirred for 30 minutes. Furthermore, 0.25 g of DCC was added, and the mixture was stirred at room temperature for 2 hours. After the reaction, the resulting DCU was removed by filtration, and the filtrate was treated to remove the solvent using an evaporator. Next, crystallization using ethyl acetate and hexane was performed three times, after which the obtained crystal was dried. 50 mg of glycyl-glycyl was added to 1 mL of water, the mixture was stirred, and 25 mg of triethylamine was added. To this solution, a solution of 2.5 g of the above-described crystal dissolved in 3 mL of acetonitrile was added drop by drop, and they were reacted at room temperature for 3 hours. After the reaction, the mixture was concentrated and dehydrated, and subjected to crystallization using ethyl acetate and hexane, and the crystal was dried to give 1.75 g of a crystal.

### (4) Synthesis of methylpolyoxyethylenecarbamyl-cysteine (methylpolyoxyethylenemaleimide)-glycyl-glycyl-distearoylphosphatidylethanolamine

1.5 g (0.2 mmol) of the methylpolyoxyethylenecarbamyl-cysteine (methylpolyoxyethylenemaleimide)-glycyl-glycyl obtained in (3) above was added to 10 mL of chloroform, and the mixture was dissolved with stirring at 40 to 45°C. Furthermore, 0.5 g (0.675 mmol) of distearoylphosphatidylethanolamine was added, and the mixture was stirred; next, 0.7 g (3.4 mmol) of dicyclohexylcarbodiimide was added, and the mixture was stirred, after which 10 mg (0.1 mmol) of triethylamine was added, and they were reacted for 4 hours. After the reaction, insoluble matter was removed by filtration, the filtrate was treated to remove the chloroform using an evaporator, subsequently the residue was re-dissolved in 5 mL of chloroform, and column separation and purification were performed under the conditions shown below. 100 g of WakogelC-100 was used as the stationary phase, and a mixed solvent of chloroform and methanol was used as the mobile phase. Fractionation was performed while the mixing ratio (ratio by volume) of chloroform /methanol was changed from 95/5 to 85/15. The obtained fraction was treated to remove the solvent, 20 mL of hexane was added to cause crystallization, the crystal was collected by filtration and dried to give 500 mg of the title compound.

The progression of the reaction and identification of the product were performed by thin-layer chromatography (TLC) in the same manner as described above except that a mixed solvent of chloroform, methanol and water (mixing ratio (ratio by volume) was 65:25:4) was used as the developing solvent. The reaction end point was confirmed by the fact that the spot of methylpolyoxyethylenecarbamyl-cysteine (methylpolyoxyethylenemaleimide)-glycyl-glycyl detected near an Rf value of 0.5 and the spot of distearoylphosphatidylethanolamine detected near an Rf value of 0.6 were converted to a spot detected near an Rf of 0.75. Regarding the identification of the product, the presence of the monomethoxyoxyethylene chain, peptide chain and distearoylphosphatidylethanolamine was confirmed by the fact that ¹H-NMR (400 MHz, CDCl₃) detected the methyl group of the terminal methoxy derived from the methoxypolyethylene glycol near δ: 3.4 ppm, the polyoxyethylene group near δ: 3.5 ppm, the methylene group derived from the peptide near 1.5 ppm, and the terminal methyl group of the acyl group derived from the distearoylphosphatidylethanolamine near δ: 0.9 ppm, respectively. By MALDI-TOF/MS (BIFLEX III: manufactured by BRUKER) analysis, the average molecular weight of the obtained title compound was determined to be 7700.

### (Example 4)

### Synthesis of methylpolyoxyethylenecarbamyl-glycyl-glycyl-glycyl-distearoylphosphatidylethanolamine (DSPE-GGG-PEG2000)

After a reaction was carried out in the same manner as Example 1 using 2 g of the methylpolyoxyethylenecarbamyl-glycyl-glycyl-glycine obtained in Example 1 (1) and 1.35 g of distearoylphosphatidylethanolamine, insoluble matter was removed by filtration, and the chloroform was removed using an evaporator. Next, the residue was dissolved in ethyl acetate, hexane was added, the mixture was cooled in ice water to cause crystallization, and the crystal was collected by filtration to give a crystal. Then, the same crystallization was performed again, hexane was added to the obtained crystal, the mixture was stirred and filtered, and the filtrate was dried to give 1.5 g of the title compound.

The progression of the reaction and identification of the product were performed in the same manner as Example 1.

### (Examples 5 to 7 and Comparative Examples 1 to 3)

### (Production of liposomes and evaluation of membrane stabilities)

After liposome solutions were prepared by the methods described below, the membrane stabilities thereof were evaluated.

### (1) Preparation of liposome solutions

Using the methylpolyoxyethylenecarbamyl-glycyl-glycyl-glycyl-dioleoylphosphatidylethanolamine (DOPE-GGG-PEG2000) obtained in Example 1, dioleylphosphatidylethanolamine (DOPE), and methylpolyoxyethylenecarbamyl-dioleoylphosphatidylethanolamine (DOPE-PEG2000), a composition in the blending ratios shown in Table 1 was weighed in an egg plant flask, and dissolved in chloroform. Next, the solvent was removed using a rotary evaporator to form a thin layer of lipid on the inside wall of the flask. After the solvent was thoroughly removed under reduced pressure, the fluorescent dye HPTS and DPX, a quencher (quenching agent) thereof, were dissolved in 2 mL of Tris buffer solution (pH 10) to obtain concentrations of 15.731 mg/mL and 12.665 mg/mL, respectively, and this 2 mL was added to the lipid thin membrane, and the mixture was dispersed using a Vortex mixer. This dispersion liquid was subjected to sizing (0.4 µm X 3 times, 0.2 µm X 3 times, 0.1 µm X 3 times, 0.1 µm (2 filters superposed) X 3 times) using polycarbonate membrane filters with various pore diameters to give liposome solutions.

**(Table 1)**

| | Liposome membrane composition | Content ratio (lipid/lipid derivative, mol) |
|---|---|---|
| Example 5 | DOPE/DOPE-GGG-PEG2000 | 100/3 |
| Example 6 | DOPE/DOPE-GGG-PEG2000 | 100/5 |
| Example 7 | DOPE/DOPE-GGG-PEG2000 | 100/10 |
| Comparative Example 1 | DOPE | 100 |
| Comparative Example 2 | DOPE/DOPE-PEG2000 | 100/3 |
| Comparative Example 3 | DOPE/DOPE-PEG2000 | 100/5 |

Each liposome dispersion was subjected to gel filtration (Sephadex G-50; physiological saline used as mobile phase), the liposome fraction eluted in void volume was collected, and this was used as the liposome solution. Regarding particle diameter, the above-described liposome solution was taken, and the diameter was measured by the dynamic light scattering method (particle size analyzer: NICOMP Model 370, manufactured by Particle Sizing System). As a result, all liposomes had a particle diameter of 95 to 115 nm. The obtained liposome solutions were allowed to stand at room temperature for 1 month. When the dispersion states of the liposome solutions after 1 month were examined, the liposome solution supplemented with the lipid DOPE alone (Comparative Example 1) was not stable and sedimentation was observed, but none of the other liposome solutions exhibited a visual change and were uniform liposome solutions.

### (2) Membrane stability test of liposomes

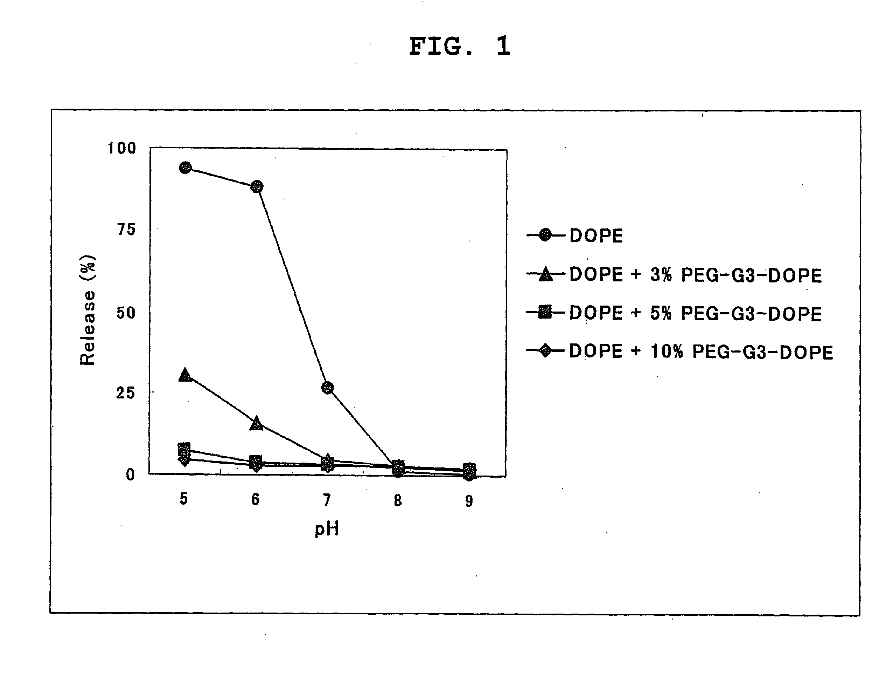

In buffer solutions at pH 5, 6, 7, 8, and 9, the liposome solutions of Examples 5 to 7 and Comparative Example 1 were incubated at 37°C for 1 hour. After the incubation, a portion was collected, added to a pH 10 buffer solution and diluted, and fluorescence intensity was measured using a measuring instrument (F-4500, manufactured by Hitachi). Then, the leakage of the fluorescent dye (HPTS) from the liposome (degree of liposome disintegration) was measured, and membrane stability was evaluated. The results are shown in Figure 1. As shown in Figure 1, when DOPE-GGG-PEG2000 was added to the liposome, no liposome disintegration due to pH differences was observed; it was confirmed that pH sensitivity was suppressed. On the other hand, in the liposome supplemented only with DOPE, because liposome disintegration was observed at pH levels lower than the neutral zone, pH sensitivity was confirmed. From this, it was confirmed that the liposome supplemented with DOPE-GGG-PEG2000 had a stability in aqueous solution increased by the polyoxyalkylene chain that constitutes the molecule thereof in ordinary state.

### (3) Stability test in blood

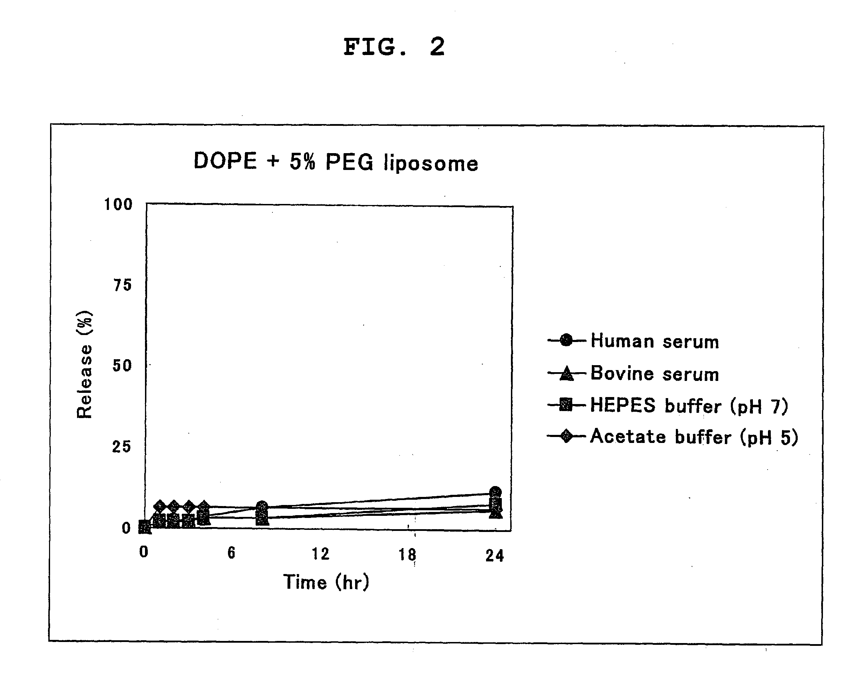

The liposomes obtained in Examples 5 to 6 and Comparative Examples 2 to 3 were incubated in serum at 37°C; after elapse of 0, 1, 3, 4, 8, and 24 hours, a portion of each thereof was collected, added to a pH 10 buffer solution and diluted. Then, fluorescence intensity was measured, whereby the leakage of the fluorescent dye (HPTS) from the liposome (degree of liposome disintegration) was measured. The measurement results for the liposome obtained in Example 6 are shown in Figure 2. Because similar results were obtained from the liposomes of Example 5 and Comparative Examples 2 to 3, it was confirmed that in human and bovine sera, the liposomes were stable, and that no liposome disintegration was observed.

### (4) Enzyme sensitivities of liposome solutions and liposome disintegratability test

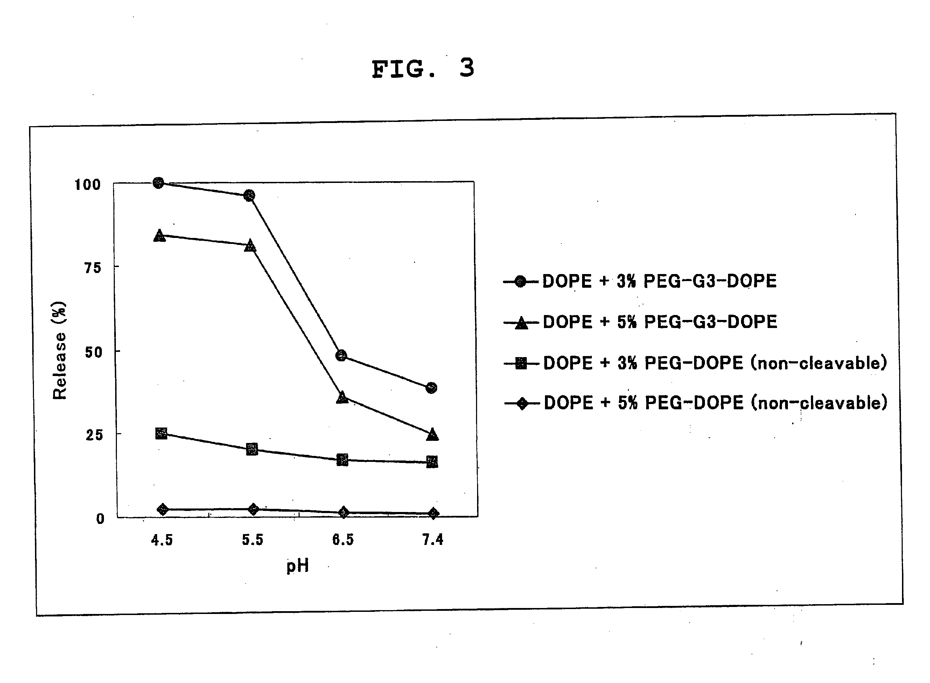

The liposomes obtained in Examples 5 to 6 and Comparative Examples 2 to 3 were incubated in the presence of papain (2 mg/mL), reduced glutathione (5 mM), and EDTA (1 mM) at 37°C for 1 hour. After completion of the incubation, a portion was collected, added to a pH 10 buffer solution and diluted, and fluorescence intensity was measured, whereby the leakage of the fluorescent dye (HPTS) from the liposome (degree of liposome disintegration) was measured. The results are shown in Figure 3. As shown in Figure 3, pH-dependent release of the included fluorescent dye was observed in the liposomes of Examples 5 to 6, but was not observed in the liposomes of Comparative Examples 2 to 3.

### (Example 8)

### Evaluation of sensitivities of liposome solutions to different enzymes and liposome disintegratability

Using DOPE alone, by the same method as (1) above, a liposome solution including a fluorescent dye whose particle diameter had been uniformized to about 100 nm was obtained. DOPE-GGG-PEG2000 dissolved in Tris buffer solution (pH 10) was added to the obtained liposome solution to obtain concentrations of 1.5, 2.5, and 5.0%, by molar ratio to the liposome; each solution was incubated at room temperature for 1 hour, and this was used as the liposome solution.

The 0.5 mM liposome solution (100 µL) thus obtained was added to a 1 M sodium acetate buffer solution (pH 5.5) containing cathepsin B (CB, 2.5 units/mL, 6.4 µM), and to the same buffer solution but not containing cathepsin B (900 µL), and they were incubated at 37°C for 1 hour and 24 hours.

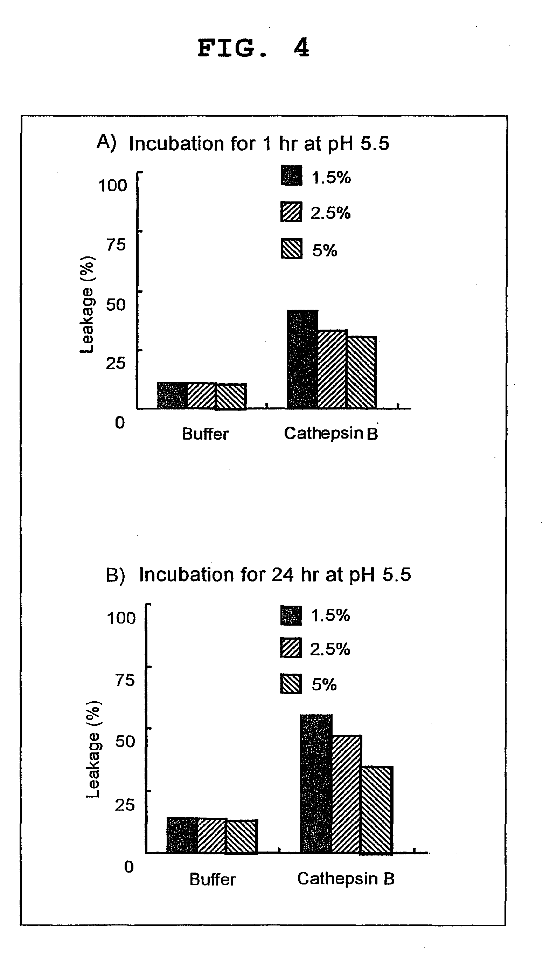

100 µL of the reaction liquid was added to 2 mL of Tris buffer solution (pH 10), the fluorescence intensity in this solution was measured by the same method as (2) above, and the degree of disintegration of the fluorescent dye from the liposome was determined. The results are shown in Figure 4. As shown in Figure 4, remarkable release of the included content was observed in the presence of the enzyme compared to the absence of the enzyme (buffer). It was confirmed that this release increased dependently to incubation time.

### (Example 9 and Comparative Examples 4 to 5)

### (1) Evaluation of blood kinetics of liposome including anticancer agent (doxorubicin) and anticancer agent retention rate of liposome

Using the methylpolyoxyethylenecarbamyl-glycyl-glycyl-glycyl-distearoylphosphatidylethanolamine (DSPE-GGG-PEG2000) obtained in Example 5 or the methylpolyoxyethylenecarbamyl-distearoylphosphatidylethanolamine (DSPE-PEG2000), egg yolk phosphatidylcholine (EPC) or hydrogenated egg yolk phosphatidylcholine (HEPC), and cholesterol, a composition in the blending ratios shown in Table 2 below was weighed in an egg plant flask, and the liposome solutions of Example 9 and Comparative Examples 4 to 5 were obtained by the same method as (1) above but using doxorubicin in place of the fluorescent dye. To monitor the blood kinetics of the liposome per se, 40 µCi/µmol ³H-cholesterylhexadecyl ether (³H-CHE) was added. Doxorubicin was included by the ammonium sulfate gradient method (0.2 mg doxorubicin/mg lipid).

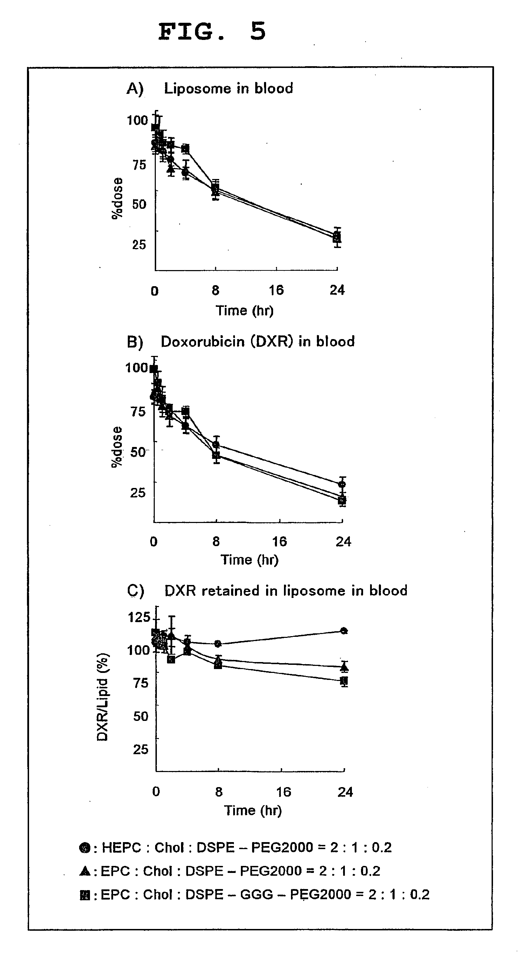

Next, each liposome was administered to a male ddY mouse from the tail vein (25 mg lipid/kg), blood was collected 2 minutes, 30 minutes, 1 hour, 2 hours, 4 hours, 8 hours, and 24 hours after the administration, the radioactivity in blood was measured to determine the concentration of the liposome per se, and doxorubicin was measured by the fluorescence method to determine the doxorubicin concentration at that time point. Because the doxorubicin that has leaked from the liposome is quickly distributed in various tissues, doxorubicin concentrations determined by this method are considered to reflect the amount of doxorubicin retained in the liposome. Hence, by determining the liposome concentration and doxorubicin concentration at each measuring point, the doxorubicin retention rate was determined. The results are shown in Figure 5. As shown in Figure 5, it was confirmed that the liposome supplemented with DSPE-GGG-PEG2000 of Example 8 exhibited a blood kinetics equivalent to that of the liposome supplemented with DSPE-PEG2000. Regarding doxorubicin retention, this liposome was equivalent to the liposome of Comparative Example 5; it was confirmed that the DSPE-GGG-PEG2000 added was minimally cleaved in blood and sufficiently protected the liposome. Furthermore, it was confirmed that in this experimental system, DSPE-GGG-PEG2000 did not influence the drug-releasing characteristics of liposome.

**(Table 2)**

| | Liposome membrane composition | Content ratio (lipid/lipid derivative, mol) |
|---|---|---|
| Example 9 | EPC/cholesterol/DSPE-GGG-PEG2000 | 20/10/2 |
| Comparative Example 4 | HEPC/cholesterol/DSPE-PEG2000 | 20/10/2 |
| Comparative Example 5 | EPC/cholesterol/DSPE-PEG2000 | 20/10/2 |

### (2) Antitumor effect of liposome including anticancer agent (doxorubicin)

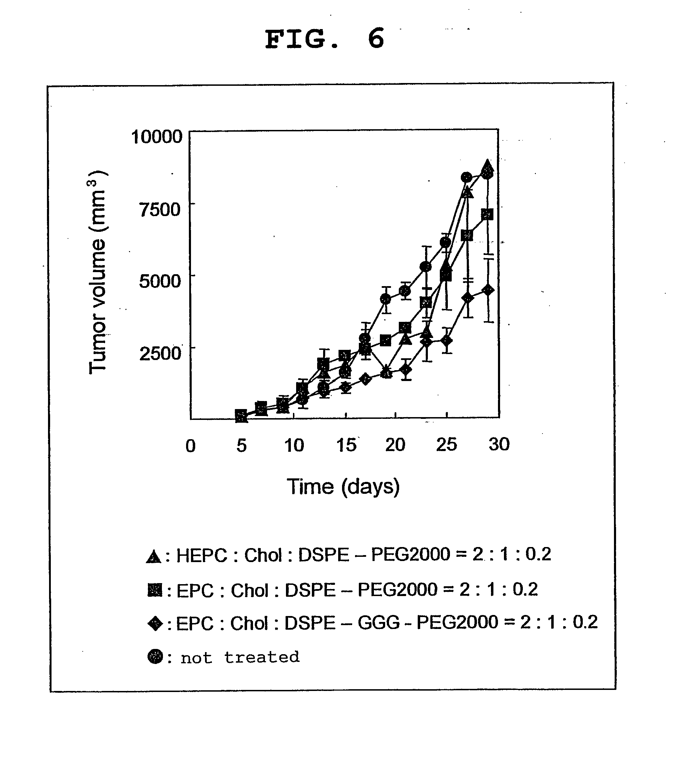

Lewis lung cancer (5X10⁵) was subcutaneously transplanted to the back of a male C57BL/6 mouse; 13 days after the transplantation, each of the doxorubicin-including liposomes of Example 9 and Comparative Examples 4 and 5 was administered from the tail vein (5 mg doxorubicin/kg), and cancer growth was monitored. The results are shown in Figure 6. As shown in Figure 6, the liposome supplemented with DSPE-GGG-PEG2000 exhibited the highest antitumor activity.

### Industrial Applicability

The lipid membrane structure of the present invention, because of the containment of the above-described lipid derivative, when applied to a drug delivery system, exhibits an improvement in stability of the lipid membrane structure, and allows efficient cleavage of the polyoxyalkylene chain at the target site, so that the retention in blood can be regulated. Furthermore, upon cleavage of the polyoxyalkylene chain, the hydrated layer covering the surface of the lipid membrane structure is lost, whereby the lipid membrane structure becomes no longer structurally stable and, in addition, exhibits pH sensitivity and other properties, resulting in the collapse of the lipid membrane structure and the release of the bioactive substance included. As a result, the selective delivery of the bioactive substance to the target site improves, which in turn can contribute to reducing adverse reactions due to overdosage.

## Claims

1. A lipid membrane structure having a lipid membrane comprising a polyoxyalkylene chain-containing lipid derivative, which is represented by the following formula (1): 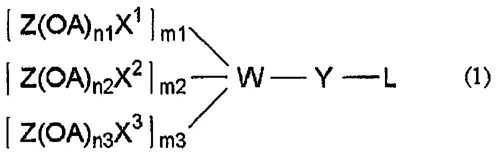 wherein
L is a residue derived from an aliphatic hydrocarbon having 8 to 24 carbon atoms, which comprises at least one kind of group out of an amino group, a hydroxyl group, a carboxyl group and a maleimide group, or a lipid selected from the group consisting of a glycerolipid, a sphingolipid and a sterol,
Y is a divalent group comprising a group selected from the group consisting of -CONH-, -NHCO-, -OCONH-, -NHOCO-, -COO-, -OOC-, -CH₂NH-, -NHCH₂-, -S-CH<, >CH-S-, and -O-,
W is a polyamino acid residue comprising 2 to 6 glycine residues, which is represented by Gly-Gly, Gly-Gly-Gly, Gly-Gly-Gly-Gly, Gly-Gly-Gly-Gly-Gly, or Gly-Gly-Gly-Gly-Gly-Gly,
X¹ is a divalent group comprising -NHCO- or -OOC-,
X² is a divalent group comprising a group selected from the group consisting of -OCONH-, -CONH-, -CH₂NH-, and - NHC(O)NH-,
X³ is a divalent group comprising a group selected from the group consisting of >CH-S-, -NHCOO-, -COO-, -COS-, and -O-, OA is an oxyalkylene group having 2 to 4 carbon atoms,
Z is a hydrogen atom or a methyl group,
each of m1, m2 and m3 independently represents a positive number of 0 to 5 that satisfies the requirement of 1 ≤ m1+m2+m3 ≤ 9, and
each of n1, n2 and n3 independently represents a positive number of 4 to 800 that satisfies the requirement of 4 ≤ (n1Xm1)+(n2Xm2)+(n3Xm3) ≤ 2000, and a phosphatidylethanolamine as a constituent of the lipid membrane.

2. The structure of claim 1, wherein X¹ is a divalent group comprising -OOC-, X² is a divalent group comprising -OCONH- or -CH₂NH-, and X³ is a divalent group comprising >CH-S- or -O-.

3. The structure of claim 2, wherein m1 is 0.

4. The structure of any one of claims 1 to 3, wherein OA is an oxyethylene group.

5. The structure of any one of claims 1 to 4, wherein each of m1, m2 and m3 is a positive number that satisfies the requirement of 1 ≤ m1+m2+m3 ≤ 6, and each of n1, n2 and n3 is a positive number that satisfies the requirement of 40≤ (n1Xm1)+(n2Xm2)+(n3Xm3) ≤ 1000.

6. The structure of any one of claims 1 to 5, wherein each of m1, m2 and m3 is a positive number that satisfies the requirement of 2 ≤ m1+m2+m3 ≤ 6.

7. The structure of any one of claims 1 to 6, wherein each of m1 and m3 is 0, m2 is 1, X² is a divalent group comprising -OCONH-, and Y is a divalent group comprising - NHCO-.

8. The structure of any one of claims 1 to 7, wherein L is a residue derived from a glycerophospholipid.

9. The structure of any one of claims 1 to 8, wherein W is a polyamino acid residue comprising a dipeptide represented by Phe-Gly, Leu-Gly, or Tyr-Gly, and/or W is a polyamino acid residue comprising a Lys residue, an Arg residue, a Cys residue, an Asp residue, a Glu residue or a Ser residue.

10. The structure of any one of claim 1 to 9, wherein the content ratio of the polyoxyalkylene chain-containing lipid derivative is 0.1 to 25% by weight relative to the total weight of the lipid membrane structure.

11. The structure of any one of claims 1 to 10, wherein the phosphatidylethanolamine is dioleoylphosphatidylethanolamine.

12. The structure of any one of claims 1 to 11, which is in the form of a liposome.

13. The structure of claim 12, comprising a bioactive substance.

14. The structure of claim 13, wherein the bioactive substance is released at pH 6 or below.

15. The structure of claim 1, wherein L is a dioleoylphosphatidylethanolamine residue, and the phosphatidylethanolamine is dioleoylphosphatidylethanolamine.

16. A polyoxyalkylene chain-containing lipid derivative, which is represented by the following formula (1): 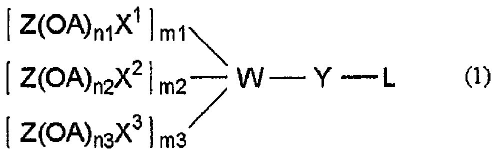 wherein
L is a residue derived from an aliphatic hydrocarbon having 8 to 24 carbon atoms, which comprises at least one kind of group out of an amino group, a hydroxyl group, a carboxyl group and a maleimide group, or a lipid selected from the group consisting of a glycerolipid, a sphingolipid and a sterol,
Y is a divalent group comprising a group selected from the group consisting of -CONH-, -NHCO-, -OCONH-, -NHOCO-, -COO-, -OOC-, -CH₂NH-, -NHCH₂-, -S-CH<, >CH-S-, and -O-,
W is a polyamino acid residue comprising 2 to 6 glycine residues, which is represented by Gly-Gly, Gly-Gly-Gly, Gly-Gly-Gly-Gly, Gly-Gly-Gly-Gly-Gly, or Gly-Gly-Gly-Gly-Gly-Gly,
X¹ is a divalent group comprising -NHCO- or -OOC-,
X² is a divalent group comprising a group selected from the group consisting of -OCONH-, -CONH-, -CH₂NH-, and - NHC(O)NH-,
X³ is a divalent group comprising a group selected from the group consisting of >CH-S-, -NHCOO-, -COO-, -COS-, and -O-, OA is an oxyalkylene group having 2 to 4 carbon atoms,
Z is a hydrogen atom or a methyl group,
each of m1, m2 and m3 independently represents a positive number of 0 to 5 that satisfies the requirement of 1 ≤ m1+m2+m3 ≤ 9, and
each of n1, n2 and n3 independently represents a positive number of 4 to 800 that satisfies the requirement of 4 < (n1Xm1) + (n2Xm2) + (n3Xm3) ≤ 2000.

## Patentansprüche

1. Lipidmembranstruktur, die eine Lipidmembran aufweist, umfassend ein polyoxyalkylenkettenhaltiges Lipidderivat, das durch die folgende Formel (1) dargestellt wird: 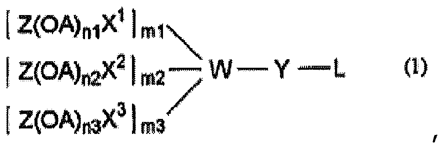 wobei
L ein von einem aliphatischen Kohlenwasserstoff mit 8 bis 24 Kohlenstoffatomen abgeleiteter Rest, der wenigstens eine Art von Gruppe umfasst, die aus einer Aminogruppe, einer Hydroxygruppe, einer Carboxygruppe und einer Maleinimidgruppe ausgewählt ist, oder ein Lipid, das aus der Gruppe ausgewählt ist, die aus einem Glycerolipid, einem Sphingolipid und einem Sterin besteht, ist;
Y eine zweiwertige Gruppe ist, die eine Gruppe umfasst, die aus der Gruppe ausgewählt ist, die aus -CONH-, -NHCO-, -OCONH-, -NHOCO-, -COO-, -OOC-, -CH₂NH-, -NHCH₂-, -S-CH<, >CH-S- und -O- besteht;
W ein Polyaminosäurerest ist, der 2 bis 6 Glycinreste umfasst und der durch Gly-Gly, Gly-Gly-Gly, Gly-Gly-Gly-Gly, Gly-Gly-Gly-Gly-Gly oder Gly-Gly-Gly-Gly-Gly-Gly dargestellt wird;
X¹ eine zweiwertige Gruppe ist, die -NHCO- oder -OOC- umfasst;
X² eine zweiwertige Gruppe ist, die eine Gruppe umfasst, die aus der Gruppe ausgewählt ist, die aus -OCONH-, -CONH-, -CH₂NH- und -NHC(O)NH- besteht;
X³ eine zweiwertige Gruppe ist, die eine Gruppe umfasst, die aus der Gruppe ausgewählt ist, die aus >CH-S-, -NHCOO-, -COO-, -COS- und -O- besteht;
OA eine Oxyalkylengruppe mit 2 bis 4 Kohlenstoffatomen ist;
Z ein Wasserstoffatom oder eine Methylgruppe ist;
m1, m2 und m3 jeweils unabhängig für eine positive Zahl von 0 bis 5 stehen, die der Bedingung 1 ≤ m1+m2+m3 ≤ 9 genügt; und
n1, n2 und n3 jeweils unabhängig für eine positive Zahl von 4 bis 800 stehen, die der Bedingung 4 ≤ (n1Xm1)+(n2Xm2)+(n3Xm3) ≤ 2000 genügt;
und ein Phosphatidylethanolamin als Bestandteil der Lipidmembran.

2. Struktur gemäß Anspruch 1, wobei X¹ eine zweiwertige Gruppe ist, die -OOC- umfasst, X² eine zweiwertige Gruppe ist, die -OCONH- oder -CH₂NH- umfasst, und X³ eine zweiwertige Gruppe ist, die >CH-S- oder -O- umfasst.

3. Struktur gemäß Anspruch 2, wobei m1 = 0 ist.

4. Struktur gemäß einem der Ansprüche 1 bis 3, wobei OA eine Oxyethylengruppe ist.

5. Struktur gemäß einem der Ansprüche 1 bis 4, wobei m1, m2 und m3 jeweils eine positive Zahl sind, die der Bedingung 1 ≤ m1+m2+m3 ≤ 6 genügt, und n1, n2 und n3 jeweils eine positive Zahl sind, die der Bedingung 40 ≤ (n1Xm1)+(n2Xm2)+(n3Xm3) ≤ 1000 genügt.

6. Struktur gemäß einem der Ansprüche 1 bis 5, wobei m1, m2 und m3 jeweils eine positive Zahl sind, die der Bedingung 2 ≤ m1+m2+m3 ≤ 6 genügt.

7. Struktur gemäß einem der Ansprüche 1 bis 6, wobei m1 und m3 jeweils = 0 sind, m2 = 1 ist, X² eine zweiwertige Gruppe ist, die -OCONH- umfasst, und Y eine zweiwertige Gruppe ist, die -NHCO- umfasst.

8. Struktur gemäß einem der Ansprüche 1 bis 7, wobei L ein von einem Glycerophospholipid abgeleiteter Rest ist.

9. Struktur gemäß einem der Ansprüche 1 bis 8, wobei W ein Polyaminosäurerest ist, der ein Dipeptid umfasst, das durch Phe-Gly, Leu-Gly oder Tyr-Gly dargestellt wird, und/oder W ein Polyaminosäurerest ist, der einen Lys-Rest, einen Arg-Rest, einen Cys-Rest, einen Asp-Rest, einen Glu-Rest oder einen Ser-Rest umfasst.

10. Struktur gemäß einem der Ansprüche 1 bis 9, wobei der Gehaltsanteil des polyoxyalkylenkettenhaltigen Lipidderivats 0,1 bis 25 Gew.-% beträgt, bezogen auf das Gesamtgewicht der Lipidmembranstruktur.

11. Struktur gemäß einem der Ansprüche 1 bis 10, wobei es sich bei dem Phosphatidylethanolamin um Dioleoylphosphatidylethanolamin handelt.

12. Struktur gemäß einem der Ansprüche 1 bis 11, die in Form eines Liposoms vorliegt.

13. Struktur gemäß Anspruch 12, die eine bioaktive Substanz umfasst.

14. Struktur gemäß Anspruch 13, wobei die bioaktive Substanz bei pH 6 oder darunter freigesetzt wird.

15. Struktur gemäß Anspruch 1, wobei L ein Dioleoylphosphatidylethanolamin-Rest ist und es sich bei dem Phosphatidylethanolamin um Dioleoylphosphatidylethanolamin handelt.

16. Polyoxyalkylenkettenhaltiges Lipidderivat, das durch die folgende Formel (1) dargestellt wird: 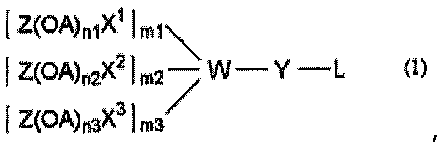 wobei
L ein von einem aliphatischen Kohlenwasserstoff mit 8 bis 24 Kohlenstoffatomen abgeleiteter Rest, der wenigstens eine Art von Gruppe umfasst, die aus einer Aminogruppe, einer Hydroxygruppe, einer Carboxygruppe und einer Maleinimidgruppe ausgewählt ist, oder ein Lipid, das aus der Gruppe ausgewählt ist, die aus einem Glycerolipid, einem Sphingolipid und einem Sterin besteht, ist;
Y eine zweiwertige Gruppe ist, die eine Gruppe umfasst, die aus der Gruppe ausgewählt ist, die aus -CONH-, -NHCO-, -OCONH-, -NHOCO-, -COO-, -OOC-, -CH₂NH-, -NHCH₂-, -S-CH<, >CH-S- und -O- besteht;
W ein Polyaminosäurerest ist, der 2 bis 6 Glycinreste umfasst und der durch Gly-Gly, Gly-Gly-Gly, Gly-Gly-Gly-Gly, Gly-Gly-Gly-Gly-Gly oder Gly-Gly-Gly-Gly-Gly-Gly dargestellt wird;
X¹ eine zweiwertige Gruppe ist, die -NHCO- oder -OOC- umfasst;
X² eine zweiwertige Gruppe ist, die eine Gruppe umfasst, die aus der Gruppe ausgewählt ist, die aus -OCONH-, -CONH-, -CH₂NH- und -NHC(O)NH- besteht;
X³ eine zweiwertige Gruppe ist, die eine Gruppe umfasst, die aus der Gruppe ausgewählt ist, die aus >CH-S-, -NHCOO-, -COO-, -COS- und -O- besteht;
OA eine Oxyalkylengruppe mit 2 bis 4 Kohlenstoffatomen ist;
Z ein Wasserstoffatom oder eine Methylgruppe ist;
m1, m2 und m3 jeweils unabhängig für eine positive Zahl von 0 bis 5 stehen, die der Bedingung 1 ≤ m1+m2+m3 ≤ 9 genügt; und
n1, n2 und n3 jeweils unabhängig für eine positive Zahl von 4 bis 800 stehen, die der Bedingung 4 ≤ (n1Xm1)+(n2Xm2)+(n3Xm3) ≤ 2000 genügt.

## Revendications

1. Structure de membrane lipidique ayant une membrane lipidique comprenant un dérivé lipidique contenant une chaîne polyoxyalkylène, qui est représenté par la formule (1) suivante : 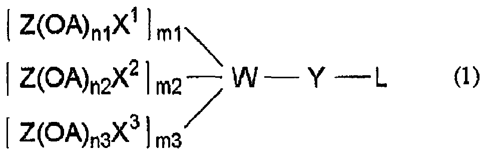 dans laquelle
L représente un résidu dérivé d'un hydrocarbure aliphatique comportant 8 à 24 atomes de carbone, qui comprend au moins une sorte de groupe parmi un groupe amino, un groupe hydroxyle, un groupe carboxyle et un groupe maléimide, ou un lipide choisi dans le groupe constitué d'un glycérolipide, un sphingolipide et un stérol,
Y représente un groupe bivalent comprenant un groupe choisi dans le groupe constitué de -CONH-, -NHCO-, -OCONH-, -NHOCO-, -COO-, -OOC-, -CH₂NH-, -NHCH₂-, -S-CH<, >CH-S-, et -O-,
W représente un résidu de polyacide aminé comprenant 2 à 6 résidus de glycine, qui est représenté par Gly-Gly, Gly-Gly-Gly, Gly-Gly-Gly-Gly, Gly-Gly-Gly-Gly-Gly, ou Gly-Gly-Gly-Gly-Gly-Gly,
X¹ représente un groupe bivalent comprenant -NHCO-ou -OOC-,
X² représente un groupe bivalent comprenant un groupe choisi dans le groupe constitué de -OCONH-, -CONH-, -CH₂NH-, et -NHC(O)NH-,
X³ représente un groupe bivalent comprenant un groupe choisi dans le groupe constitué de >CH-S-, -NHCOO-, -COO-, -COS-, et -O-,
OA représente un groupe oxyalkylène comportant 2 à 4 atomes de carbone,
Z représente un atome d'hydrogène ou un groupe méthyle,
chacun de m1, m2 et m3 représente indépendamment un nombre positif de 0 à 5 qui satisfait l'exigence de 1 ≤ m1+m2+m3 ≤ 9, et
chacun de n1, n2 et n3 représente indépendamment un nombre positif de 4 à 800 qui satisfait l'exigence de 4 ≤ (n1Xm1)+(n2Xm2)+(n3Xm3) ≤ 2000,
et une phosphatidyléthanolamine comme constituant de la membrane lipidique.

2. Structure selon la revendication 1, dans laquelle X¹ représente un groupe bivalent comprenant -OOC-, X² représente un groupe bivalent comprenant -OCONH- ou -CH₂NH-, et X³ représente un groupe bivalent comprenant >CH-S- ou -0-.

3. Structure selon la revendication 2, dans laquelle m1 vaut 0.

4. Structure selon l'une quelconque des revendications 1 à 3, dans laquelle OA représente un groupe oxyéthylène.

5. Structure selon l'une quelconque des revendications 1 à 4, dans laquelle chacun de m1, m2 et m3 représente un nombre positif qui satisfait l'exigence de 1 ≤ m1+m2+m3 ≤ 6, et chacun de n1, n2 et n3 représente un nombre positif qui satisfait l'exigence de 40 ≤ (n1Xm1)+(n2Xm2)+(n3Xm3) ≤ 1000.

6. Structure selon l'une quelconque des revendications 1 à 5, dans laquelle chacun de m1, m2 et m3 représente un nombre positif qui satisfait l'exigence de 2 ≤ m1+m2+m3 ≤ 6.

7. Structure selon l'une quelconque des revendications 1 à 6, dans laquelle chacun de m1 et m3 vaut 0, et m2 vaut 1, X² représente un groupe bivalent comprenant -OCONH-, et Y représente un groupe bivalent comprenant -NHCO-.

8. Structure selon l'une quelconque des revendications 1 à 7, dans laquelle L représente un résidu dérivé d'un glycérophospholipide.

9. Structure selon l'une quelconque des revendications 1 à 8, dans laquelle W représente un résidu de polyacide aminé comprenant un dipeptide représenté par Phe-Gly, Leu-Gly, ou Tyr-Gly, et/ou W représente un résidu de polyacide aminé comprenant un résidu Lys, un résidu Arg, un résidu Cys, un résidu Asp, un résidu Glu ou un résidu Ser.

10. Structure selon l'une quelconque des revendications 1 à 9, dans laquelle le rapport de la teneur du dérivé lipidique contenant une chaîne polyoxyalkylène est de 0,1 à 25 % en poids par rapport au poids total de la structure de membrane lipidique.

11. Structure selon l'une quelconque des revendications 1 à 10, dans laquelle la phosphatidyléthanolamine est la dioléoylphosphatidyléthanolamine.

12. Structure selon l'une quelconque des revendications 1 à 11, qui est sous la forme d'un liposome.

13. Structure selon la revendication 12, comprenant une substance bioactive.

14. Structure selon la revendication 13, dans laquelle la substance bioactive est libérée à pH 6 ou inférieur.

15. Structure selon la revendication 1, dans laquelle L représente un résidu de dioléoylphosphatidyléthanolamine, et la phosphatidyléthanolamine est la dioléoylphosphatidyléthanolamine.

16. Dérivé lipidique contenant une chaîne polyoxyalkylène, qui est représenté par la formule (1) suivante : 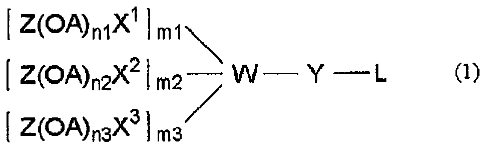 dans laquelle
L représente un résidu dérivé d'un hydrocarbure aliphatique comportant 8 à 24 atomes de carbone, qui comprend au moins une sorte de groupe parmi un groupe amino, un groupe hydroxyle, un groupe carboxyle et un groupe maléimide, ou un lipide choisi dans le groupe constitué d'un glycérolipide, un sphingolipide et un stérol,
Y représente un groupe bivalent comprenant un groupe choisi dans le groupe constitué de -CONH-, -NHCO-, -OCONH-, -NHOCO-, -COO-, -OOC-, -CH₂NH-, -NHCH₂-, -S-CH<, >CH-S-, et -O-,
W représente un résidu de polyacide aminé comprenant 2 à 6 résidus de glycine, qui est représenté par Gly-Gly, Gly-Gly-Gly, Gly-Gly-Gly-Gly, Gly-Gly-Gly-Gly-Gly, ou Gly-Gly-Gly-Gly-Gly-Gly,
X¹ représente un groupe bivalent comprenant -NHCO3-ou -OOC-,
X² représente un groupe bivalent comprenant un groupe choisi dans le groupe constitué de -OCONH-, -CONH-, -CH₂NH-, et -NHC(O)NH-,
X³ représente un groupe bivalent comprenant un groupe choisi dans le groupe constitué de >CH-S-, -NHCOO-, -COO-, -COS-, et -O-,
OA représente un groupe oxyalkylène comportant 2 à 4 atomes de carbone,
Z représente un atome d'hydrogène ou un groupe méthyle,
chacun de m1, m2 et m3 représente indépendamment un nombre positif de 0 à 5 qui satisfait l'exigence de 1 ≤ m1+m2+m3 ≤ 9, et
chacun de n1, n2 et n3 représente indépendamment un nombre positif de 4 à 800 qui satisfait l'exigence de 4 ≤ (n1Xm1) + (n2Xm2) + (n3Xm3) ≤ 2000.


### SEQUENCE LISTING

<110> The University of Tokushima NOF Corporation
<120> Polyoxyalkylene chain-containing lipid derivative and lipid film structure containing such derivative
<130> N102403A
<150> JP 2005-043196
   <151> 2005-02-18
<160> 11
<170> PatentIn version 3.4
<210> 1
   <211> 4
   <212> PRT
   <213> Artificial
<220>
   <223> A part of a lipid derivative
<400> 1
   Gly Gly Gly Gly
   1
<210> 2
   <211> 5
   <212> PRT
   <213> Artificial
<220>
   <223> A part of a lipid derivative
<400> 2
   Gly Gly Gly Gly Gly
   1 5
<210> 3
   <211> 6
   <212> PRT
   <213> Artificial
<220>
   <223> A part of a lipid derivative
<400> 3
   Gly Gly Gly Gly Gly Gly
   1 5
<211> 4
   <212> PRT
   <213> Artificial
<220>
   <223> A part of a lipid derivative
<400> 4
<210> 5
   <211> 4
   <212> PRT
   <213> Artificial
<220>
   <223> A part of a lipid derivative
<400> 5
   Gly Phe Gly Gly
   1
<210> 6
   <211> 4
   <212> PRT
   <213> Artificial
<220>
   <223> A part of a lipid derivative
<400> 6
   Phe Gly Gly Gly
   1
<210> 7
   <211> 4
   <212> PRT
   <213> Artificial
<220>
   <223> A part of a lipid derivative
<400> 7
   Phe Phe Gly Gly
   1
<210> 8
   <211> 5
   <212> PRT
   <213> Artificial
<220>
   <223> A part of a lipid derivative
<400> 8
   Gly Gly Gly Phe Gly
   1               5
<210> 9
   <211> 4
   <212> PRT
   <213> Artificial
<220>
   <223> A part of a lipid derivative
<400> 9
   Gly Gly Phe Phe
   1
<210> 10
   <211> 4
   <212> PRT
   <213> Artificial
<220>
   <223> A part of a lipid derivative
<400> 10
   Gly Gly Gly Phe
   1
<210> 11
   <211> 5
   <212> PRT
   <213> Artificial
<220>
   <223> A part of a lipid derivative
<220>
   <221> misc_feature
   <222> (1)..(1)
   <223> Xaa is a glycyl residue
<220>
   <221> misc_feature
   <222> (3)..(4)
   <223> Xaa is a glycyl residue
<400> 11
   Xaa Lys Xaa Xaa Gly
   1               5